# FASCICULE DE BREVET EUROPEEN

(11) **EP 2 231 598 B1**
(45) Date de publication et mention de la délivrance du brevet: **17.08.2011**
(21) Numéro de dépôt: 08872697.1
(22) Date de dépôt: 10.12.2008
(51) Int. Cl.: C07D 209/08, A61K 31/404

(54) **NOUVEAUX DERIVES DIAZENIUMDIOLATES, LEUR PROCEDE DE PREPARATION ET LES COMPOSITIONS PHARMACEUTIQUES QUI LES CONTIENNENT**
Neue Diazeniumdiolat-Derivate, ihr Herstellungsverfahren und sie enthaltende pharmazeutische Zusammensetzungen
NOVEL DIAZENIUMDIOLATE DERIVATIVES, METHOD FOR THE PREPARATION THEREOF AND PHARMACEUTICAL COMPOSITIONS CONTAINING THE SAME

(30) Priorité: 11.12.2007 FR 0708604
(43) Date de publication de la demande: 29.09.2010
(73) Titulaire: Les Laboratoires Servier, 92284 Suresnes Cedex (FR)
(72) Inventeur: CORDI Alexis, F-92150 Suresnes (FR); HABERKORN Laure, F-92270 Bois-Colombes (FR); VERBEUREN Tony, F-78540 Vernoillet (FR); COURCHAY Christine, F-91430 Igny (FR); SIMONET Serge, F-78700 Conflans-Ste-Honorine (FR)
(74) Mandataire: Giudicelli, Cathy
(86) Numéro de dépôt international: PCT/FR2008/001716
(87) Numéro de publication internationale: WO 2009/103875

(56) Documents cités:
- WO-A-98/19996
- FR-A- 2 003 311
- JP-A- 7 118 231
- JP-A- 58 124 766
- LIU D-G ET AL: "Acylsulfonamide-containing PTP1B inhibitors designed to mimic an enzyme-bound water of hydration" BIOORGANIC & MEDICINAL CHEMISTRY LETTERS, OXFORD, GB, vol. 13, no. 18, 2003, pages 3005-3007, XP002337095 ISSN: 0960-894X
- KONTER, JOERG ET AL: "Synthesis of diazen-1-ium-1,2-diolates monitored by the "NOtizer" apparatus: relationship between formation rates, molecular structure and the release of nitric oxide" EUROPEAN JOURNAL OF ORGANIC CHEMISTRY , (4), 616 -624 CODEN: EJOCFK; ISSN: 1434-193X, 2007, XP002473169
- SAAVEDRA, JOSEPH E. ET AL: "Piperazine as a linker for incorporating the nitric oxide-releasing diazeniumdiolate group into other biomedically relevant functional molecules" JOURNAL OF ORGANIC CHEMISTRY , 64(14), 5124 -5131 CODEN: JOCEAH; ISSN: 0022-3263, 1999, XP002473170

## Description

La présente invention concerne de nouveaux dérivés diazéniumdiolates, leur procédé de préparation et les compositions pharmaceutiques qui les contiennent.

Ces composés présentent des structures originales et trouvent leur application dans le domaine de l'hypertension et des maladies cardiovasculaires.

L'hypertension entraîne un risque élevé d'accidents vasculaires, en particulier au niveau cérébral et coronaire. Elle est de plus en plus fréquemment associée à d'autres pathologies comme l'athérosclérose ou des maladies métaboliques telles que l'obésité, le diabète ou l'insuffisance rénale, ce qui augmente considérablement le risque de spasmes et de thromboses.

Les diurétiques représentent une classe de médicaments antihypertenseurs très efficaces. Leur principal effet est de réduire la résistance périphérique totale via une augmentation de l'excrétion de sodium et une diminution du volume plasmatique. Les diurétiques diminuent le nombre d'accidents cardiovasculaires et sont également efficaces dans l'insuffisance cardiaque. Ils ont de plus l'avantage d'un faible nombre de contre-indications et d'une bonne tolérance. Ils peuvent être associés aux autres classes d'antihypertenseurs et sont systématiquement inclus lorsqu'une bi- ou tri-thérapie s'impose. Des dérivés indoles ont été décrits comme antihypertenseurs dans les demandes JP 07 118231, JP 58 124766 et FR 2 003 311, ou antidiabétique dans Liu et al (Bio. Med. Chem. Lett. 2003, 13, 3005-7).

Depuis la découverte en 1980 de son action cardiovasculaire, le monoxyde d'azote (NO) est reconnu comme une molécule vasodilatatrice et vasoprotectrice capable de prévenir les vasospasmes, l'athérosclérose et la thrombose, ce médiateur endogène offrant ainsi une protection contre les maladies cardiovasculaires. Le NO est essentiellement produit par les cellules endothéliales, et dans les pathologies cardiovasculaires, un dysfonctionnement de l'endothélium provoque une déficience en NO endogène.

Des composés nitrovasodilatateurs, tels que la nitroglycérine, sont utilisés depuis longtemps pour soigner l'angine de poitrine et l'insuffisance cardiaque. L'effet bénéfique de ces produits est lié à leur capacité de former (de façon spontanée ou de façon métabolique) du NO. L'utilisation de ces produits a aussi conduit à l'observation que chez le sujet hypertendu, ces donneurs de NO provoquent une baisse prédominante de la pression artérielle systolique. Une pression artérielle systolique non contrôlée est un facteur de risque important pour les accidents cérébraux et cardiaques et elle est souvent résistante aux traitements des antihypertenseurs. En effet, malgré des effets démontrés antihypertenseurs et vasoprotecteurs des diurétiques et d'autres classes de produits antihypertenseurs, la pression artérielle, en particulier systolique, reste difficile à contrôler et les taux de morbidité et de mortalité restent élevés.

Ajouter à des produits diurétiques une propriété donneur de NO améliorerait leurs propriétés antihypertensives, cardio- et vasculo-protectrices et ajouterait une action anti-thrombotique directe, le NO ayant un effet anti-agrégant plaquettaire et anti-thrombotique (Walford G. et al., 2003, J. Thromb. Haemost., 1, 2112-2118). De plus, plusieurs dérivés donneurs de NO ont été décrits dans Konter et al. (Eur. J. Org. Chem. 2007, 616-24), dans Saavedra et al (J. Org. Chem. 1999, 64, 5124-31) et dans la demande de brevet WO 98/19996.

Les composés de la présente invention, outre leur originalité structurale, présentent cette double activité pharmacologique leur conférant des propriétés tout à fait surprenantes et intéressantes dans le domaine de l'hypertension et des pathologies cardiovasculaires.

Plus spécifiquement, la présente invention concerne les composés de formule (1) : 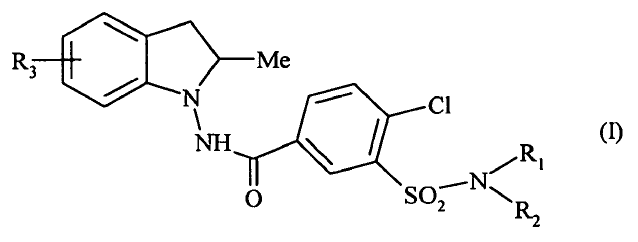 dans laquelle :
- R₁: représente un atome d'hydrogène ou un groupement -COOR dans lequel R représente un groupement alkyle (C₁-C₆) linéaire ou ramifié ou arylalkyle (C₁-C₆) linéaire ou ramifié,
- R₂: représente un groupement G ou un groupement alkyle (C₁-C₆) linéaire ou ramifié substitué par un groupement G, où G représente un groupement -(CH₂)ₙ-A-(CH₂)ₘ-B-(CR₄R₅)ₚ-(CH₂)ₒ-R₆ dans lequel :
- n vaut 0, 1, 2 ou 3,
- m vaut 0, 1, 2 ou 3,
- p vaut 0 ou 1,
- o vaut 0, 1 ou 2,
- R₄ et R₅,: identiques ou différents, représentent chacun un atome d'hydrogène ou un groupement alkyle (C₁-C₆) linéaire ou ramifié, étant entendu qu'un des groupements -CH₂- ou -CR₄R₅- de la chaîne G peut également être remplacé, si on le souhaite, par un groupement phénylène, -PhC(O)- ou -PhC(O)O- (où Ph signifie phényle),
- A et B, identiques ou différents, représentent chacun une liaison, -NH- ou un groupement : 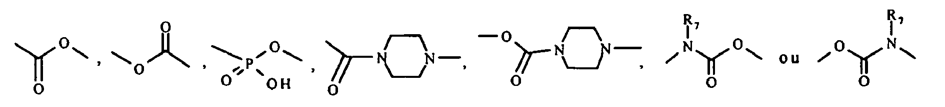 dans lesquels R₇ représente un atome d'hydrogène ou un groupement alkyle (C₁-C₆) linéaire ou ramifié,
- et R₆ représente un groupement 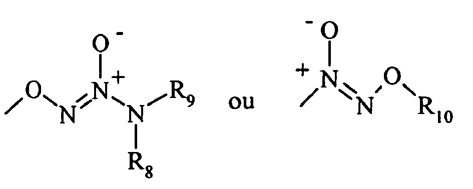 où R₈, R₉ et R₁₀, identiques ou différents, représentent chacun un atome d'hydrogène ou un groupement alkyle (C₁-C₆) linéaire ou ramifié non substitué ou substitué par un groupement amino, ou R₈ et R₉ forment ensemble une chaîne alkylène (C₁-C₆) linéaire ou ramifiée,
- R₃: représente un atome d'hydrogène, un groupement alkyle (C₁-C₆) linéaire ou ramifié ou un groupement NO₂,
leurs énantiomères et diastéréoisomères ainsi que leurs sels d'addition à un acide ou à une base pharmaceutiquement acceptable.

Parmi les acides pharmaceutiquement acceptables, on peut citer, à titre non limitatif, les acides chlorhydrique, bromhydrique, sulfurique, phosphonique, acétique, trifluoroacétique, lactique, pyruvique, malonique, succinique, glutarique, fumarique, tartrique, maléique, citrique, ascorbique, méthane sulfonique, camphorique, etc...

Parmi les bases pharmaceutiquement acceptables, on peut citer, à titre non limitatif, l'hydroxyde de sodium, l'hydroxyde de potassium, la triéthylamine, la tertbutylamine, etc...

Plus particulièrement, l'invention concerne les composés de formule (I) pour lesquels R₁ représente un atome d'hydrogène.

Avantageusement, l'invention concerne les composés de formule (I) pour lesquels R₃ représente un atome d'hydrogène ou un groupement NO₂.

R₂ représente avantageusement un groupement : 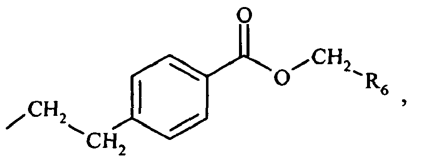 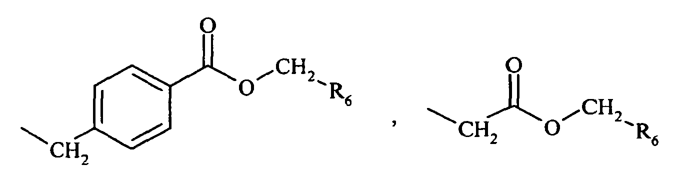 ou ―CH₂―R₆,
où R₆ est tel que défini précédemment.

Le groupement R₆ préféré selon l'invention est le groupement -O-N=N(O)-NR₈R₉. La liaison -N=N- du groupement R₆ est préférentiellement de configuration *Z*.

R₈ et R₉ représentent préférentiellement un groupement alkyle (C₁-C₆) linéaire, comme, par exemple, le groupement éthyle.

Encore plus particulièrement, l'invention concerne les composés de formule (I) qui sont :
- le 3-{[({2-chloro-5-[(2-méthyl-5-nitro-2,3-dihydro-1*H-*indol-1-yl)carbamoyl] phényl}sulfonyl)amino]méthyl}benzoate de ({[(1*Z*)-2,2-diéthyl-1-oxidohydrazono] amino}oxy)méthyle,
- le 3-{[({2-chloro-5-[(2-méthyl-2,3-dihydro-1*H-*indol-1-yl)carbamoyl]phényl} sulfonyl)amino]méthyl}benzoate de ({[(1*Z*)-2,2-diéthyl-1-oxidohydrazono]amino} oxy)méthyle,
- le ({2-chloro-5-[(2-méthyl-2,3-dihydro-1*H-*indol-1-yl)carbamoyl]phényl}sulfonyl) [({[(1*Z*)-2,2-diéthyl-1-oxidohydrazono]amino}oxy)méthyl]carbamate de *tert-*butyle,
- le *N-*(*tert*-butoxycarbonyl)-*N-*({2-chloro-5-[(2-méthyl-2,3-dihydro-1*H-*indol-1-yl) carbamoyl]phényl}sulfonyl)glycinate de ({[(1*Z*)-2,2-diéthyl-1-oxidohydrazono] amino}oxy)méthyle,
- le *N-*({2-chloro-5-[(2-méthyl-2,3-dihydro-1*H-*indol-1-yl)carbamoyl]phényl} sulfonyl)glycinate de ({[(1*Z*)-2,2-diéthyl-1-oxidohydrazono]amino}oxy)méthyle,
- le 4-{[({2-chloro-5-[(2-méthyl-2,3-dihydro-1*H-*indol-1-yl)carbamoyl]phényl} sulfonyl)amino]méthyl}benzoate de ({[(1*Z*)-2,2-diéthyl-1-oxidohydrazono]amino} oxy)méthyle,
- le 4-({[(2-chloro-5-{[(2*R*)-2-méthyl-2,3-dihydro-1*H-*indol-1-yl]carbamoyl}phényl) sulfonyl]amino}méthyl)benzoate de ({[(1*Z*)-2,2-diéthyl-1-oxidohydrazono]amino} oxy)méthyle,
- le 4-{2-[({2-chloro-5-[(2-méthyl-2,3-dihydro-1*H-*indol-1-yl)carbamoyl]phényl} sulfonyl)amino]éthyl}benzoate de ({[(1*Z*)-2,2-diéthyl-1-oxidohydrazono]amino} oxy)méthyle,
- le 4-{2-[({2-chloro-5-[((2*R*)-2-méthyl-2,3-dihydro-1*H-*indol-1-yl)carbamoyl] phényl}sulfonyl)amino]éthyl}benzoate de ({[(1*Z*)-2,2-diéthyl-1-oxidohydrazono] amino}oxy)méthyle.

L'invention concerne également le procédé de préparation des composés de formule (I) caractérisé en ce que l'on utilise comme produit de départ le composé de formule (II) : 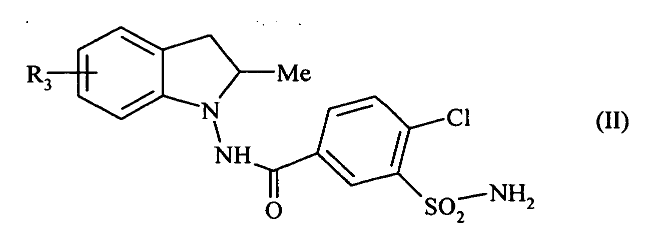 dans laquelle R₃ est tel que défini dans la formule (I),
sur lequel on condense un composé de formule (III) : 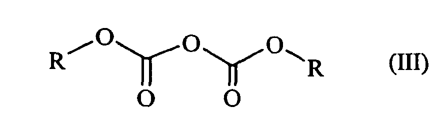 dans laquelle R est tel que défini dans la formule (I),
pour conduire au composé de formule (IV) : 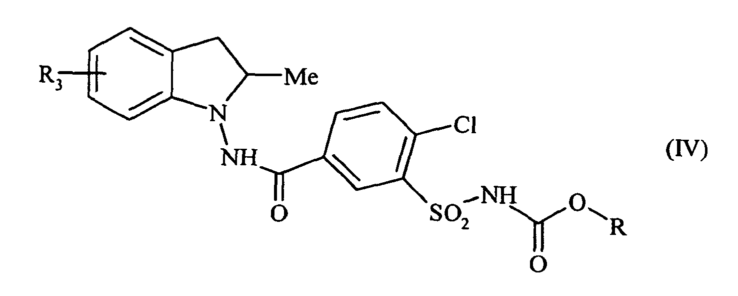 dans laquelle R₃ et R sont tels que définis précédemment,
sur lequel on condense, en présence d'une base, le composé de formule (V) :

X—R₂ (V)

dans laquelle R₂ est tel que défini dans la formule (I), et X représente un atome d'halogène, pour conduire au composé de formule (I/a), cas particulier des composés de formule (I) : 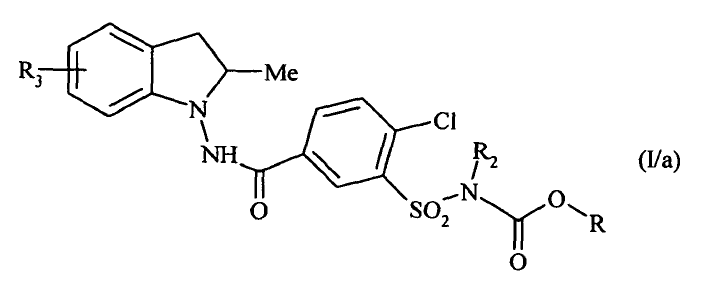 dans laquelle R, R₂ et R₃ sont tels que définis dans la formule (I),
les composés de formule (I/a) pouvant être aussi obtenus par condensation d'un composé de formule Cl-(CH₂)ₒ-R₆, dans laquelle o et R₆ sont tels que définis dans la formule (I), sur une fonction carboxylique ou phosphoramidique présente dans le groupement G,
composés de formule (I/a) qui sont optionnellement chauffés en milieu acide pour conduire au composé de formule (I/b), cas particulier des composés de formule (1) : 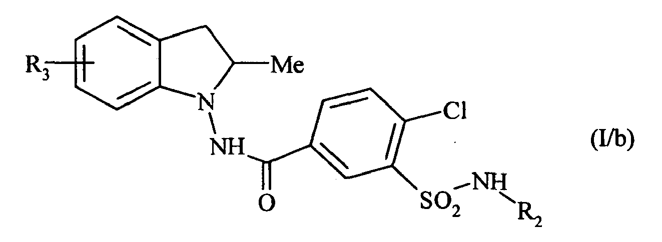 dans laquelle R₂ et R₃ sont tels que définis dans la formule (I),
les composés de formule (I/a) et (I/b) formant l'ensemble des composés de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

Les composés de formule (II) tels que définis précédemment, sont obtenus par des réactions classiques de la chimie organique, comme par exemple le procédé décrit dans le brevet FR 2 003 311.

Les composés de la présente invention, de par leurs propriétés pharmacologiques sont utiles dans le traitement de l'hypertension et des pathologies cardiovasculaires et de ses complications telles que la rétinopathie, les accidents cérébraux, la démence, l'hypertrophie ventriculaire gauche, l'insuffisance cardiaque, l'angine de poitrine, l'infarctus du myocarde et la néphropathie. Les composés selon l'invention sont également utiles dans les pathologies cardiovasculaires associées à l'athérothrombose telles que les accidents cérébraux et coronaires, les artérites et les vasculopathies ainsi que dans les complications vasculaires de nombre de maladies telles que le diabète, l'obésité, le syndrome métabolique, le cancer, la fibrose du foie etc. Les composés sont aussi utiles dans les hypertensions d'origine pulmonaire, oculaire ou portale.

La présente invention a également pour objet les compositions pharmaceutiques renfermant comme principe actif au moins un composé de formule (I), un de ses isomères optiques, ou un de ses sels d'addition à un acide ou à une base pharmaceutiquement acceptable, seul ou en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire, ou sous-cutanée), per ou trans-cutanée, intravaginale, rectale, nasale, perlinguale, buccale, oculaire ou respiratoire.

Les compositions pharmaceutiques selon l'invention pour les injections parentérales comprennent notamment les solutions stériles aqueuses et non aqueuses, les dispersions, les suspensions ou émulsions ainsi que les poudres stériles pour la reconstitution des solutions ou des dispersions injectables.

Les compositions pharmaceutiques selon l'invention, pour les administrations orales solides, comprennent notamment les comprimés simples ou dragéifiés, les comprimés sublinguaux, les sachets, les gélules, les granules, et pour les administrations liquides orales, nasales, buccales ou oculaires, comprennent notamment les émulsions, les solutions, les suspensions, les gouttes, les sirops et les aérosols.

Les compositions pharmaceutiques pour l'administration rectale ou vaginale sont préférentiellement des suppositoires, et celles pour l'administration per ou trans-cutanée comprennent notamment les poudres, les aérosols, les crèmes, les pommades, les gels et les patchs.

Les compositions pharmaceutiques citées précédemment illustrent l'invention mais ne la limitent en aucune façon.

Parmi les excipients ou véhicules inertes, non toxiques, pharmaceutiquement acceptables, on peut citer à titre indicatif et non limitatif les diluants, les solvants, les conservateurs, les agents mouillants, les émulsifiants, les agents dispersants, les liants, les agents gonflants, les agents désintégrants, les retardants, les lubrifiants, les absorbants, les agents de suspension, les colorants, les aromatisants, etc...

La posologie utile varie selon l'âge et le poids du patient, la voie d'administration, la composition pharmaceutique utilisée, la nature et la sévérité de l'affection, et la prise de traitements associés éventuels. La posologie s'échelonne de 0,1 mg à 1 g en une ou plusieurs prises par jour.

Les Exemples suivants illustrent l'invention mais ne la limitent en aucune façon. Les produits de départ utilisés sont des produits connus ou préparés selon des modes opératoires connus.

Les structures des composés, décrits dans les exemple, ont été déterminées selon les techniques spectrophotométriques usuelles (infrarouge, RMN, spectrométrie de massue...).

Les préparations suivantes conduisent à des intermédiaires de synthèse utiles dans la préparation des composés de l'invention.

### Intermédiaire 1 : Acide 5-(benzyloxy)-5-oxopentanoïque

Solubiliser l'anhydride glutarique (8,76.10⁻² mol) dans le dichlorométhane (300 ml) et placer sous agitation. Ajouter de la 4-diméthylaminopyridine (7,88.10⁻² mol) et de l'alcool benzylique (7,88.10⁻² mol) puis laisser le milieu réactionnel à température ambiante. Après 4 heures et 30 minutes, le milieu est hydrolysé avec une solution aqueuse à 5 % de carbonate de sodium (200 ml). Séparer les deux phases par décantation. La phase aqueuse est ensuite acidifiée avec une solution aqueuse 1 M d'acide chlorhydrique puis extraite avec de l'acétate d'éthyle. La phase organique est lavée avec de la saumure, séchée sur sulfate de magnésium, filtrée et évaporée à sec sous pression réduite.

Le produit du titre est obtenu sous la forme d'un solide blanc utilisé sans purification ultérieure.

### Intermédiaire 2 : Pentanedioate de benzyle et de chlorométhyle

Solubiliser l'intermédiaire 1 (2,24.10⁻² mol) dans le dichlorométhane (75 ml), ajouter de l'eau (75 ml) puis refroidir à 0 °C en agitant vigoureusement. Ajouter ensuite de l'hydrogénocarbonate de sodium (8,91.10⁻² mol) et du sulfate de tétrabutylammonium (2,24.10⁻³ mol). Après 15 minutes, additionner goutte à goutte une solution de chlorométhylchlorosulfate (2,70.10⁻² mol) dans le dichlorométhane (20 ml). Laisser le milieu réactionnel sous agitation forte à 0 °C pendant 1 heure puis laisser revenir à température ambiante. Après 1 heure et 30 minutes, les deux phases sont séparées par décantation. La phase organique est lavée avec de la saumure, séchée sur sulfate de magnésium, filtrée puis évaporée à sec sous pression réduite.

Le produit du titre est obtenu sous la forme d'une huile incolore utilisée sans purification ultérieure.

### Intermédiaire 3 : Pentanedioate de benzyle et de iodométhyle

Solubiliser de l'iodure de sodium (2,70.10⁻² mol) dans de l'acétone (100 ml). Placer sous agitation puis ajouter une solution de l'intermédiaire 2 (2,25.10⁻² mol) dans l'acétone (40 ml). Chauffer le milieu réactionnel à 45 °C. Après 48 heures, le milieu réactionnel est filtré. Le résidu solide est rincé à l'acétone puis le filtrat est évaporé à sec sous pression réduite.

Le produit du titre est obtenu sous la forme d'un résidu visqueux marron utilisé sans purification ultérieure.

### Intermédiaire 4 : Acide 4-(benzyloxy)-4-oxobutanoïque

Solubiliser l'anhydride succinique (0,10 mol) dans le dichlorométhane (300 ml) et placer sous agitation. Ajouter de la 4-diméthylaminopyridine (8,3.10⁻² mol) et de l'alcool benzylique (8,3.10⁻² mol) puis laisser le milieu réactionnel à température ambiante. Après 1 heure et 30 minutes, le milieu est hydrolysé avec une solution aqueuse à 5 % de carbonate de sodium (200 ml). Séparer les deux phases par décantation. La phase aqueuse est ensuite acidifiée avec une solution aqueuse 1 M d'acide chlorhydrique puis extraite avec de l'acétate d'éthyle. La phase organique est lavée avec de la saumure, séchée sur sulfate de magnésium, filtrée et évaporée à sec sous pression réduite.

Le produit du titre est obtenu sous la forme d'un solide blanc utilisé sans purification ultérieure.

### Intermédiaire 5 : Butanedioate de benzyle et de chlorométhyle

Solubiliser l'intermédiaire 4 (4,80.10⁻³ mol) dans le dichlorométhane (15 ml), ajouter de l'eau (15 ml) puis refroidir à 0 °C en agitant vigoureusement. Ajouter ensuite de l'hydrogénocarbonate de sodium (1,90.10⁻² mol) et du sulfate de tétrabutylammonium (4,80.10⁻⁴ mol). Après 15 minutes, additionner goutte à goutte une solution de chlorométhylchlorosulfate (5,76.10⁻³ mol) dans le dichlorométhane (4 ml). Laisser le milieu réactionnel sous agitation forte à 0 °C pendant 1 heure puis laisser revenir à température ambiante. Après 5 heures et 30 minutes, les deux phases sont séparées par décantation. La phase organique est lavée avec de la saumure, séchée sur sulfate de magnésium, filtrée puis évaporée à sec sous pression réduite. Le brut huileux incolore est chromatographié sur une colonne de silice en éluant avec un mélange *n-*heptane/acétate d'éthyle 80/20.

Le produit du titre est obtenu sous la forme d'une huile incolore.

### Intermédiaire 6 : Butanedioate de benzyle et de iodométhyle

Solubiliser de l'iodure de sodium (4,67.10⁻³ mol) dans de l'acétone (18 ml). Placer sous agitation puis ajouter une solution de l'intermédiaire 5 (3,89.10⁻³ mol) dans l'acétone (7,5 ml). Chauffer le milieu réactionnel à 45 °C. Après 6 heures, le milieu réactionnel est filtré. Le résidu solide est rincé à l'acétone puis le filtrat est évaporé à sec sous pression réduite.

Le produit du titre est obtenu sous la forme d'un résidu visqueux marron utilisé sans purification ultérieure.

### Intermédiaire 7 : 2-(Bromométhyl)benzoate de méthyle

Solubiliser le 2-méthylbenzoate de méthyle (3,33.10⁻² mol) dans le chloroforme (30 ml). Ajouter le N-bromosuccinimide (3,50.10⁻² mol), le 2,2'-azobis(2-méthylpropionitrile) (3,65.10⁻⁴ mol) et chauffer progressivement jusqu'à 65 °C pour permettre l'initiation de la réaction radicalaire. Chauffer ensuite le milieu réactionnel au reflux pendant 5 heures. Le milieu est ensuite refroidi à température ambiante. Filtrer le précipité formé. Evaporer à sec le filtrat sous pression réduite.

Le produit du titre est obtenu sous la forme d'une huile orangée utilisée sans purification ultérieure.

### Intermédiaire 8 : 4-chloro-N-[(2R)-2-méthyl-2,3-dihydro-1H-indol-1-yl]-3-sulfamoyl benzamide

Le produit du titre est obtenu après une séparation effectuée sur le 3-(aminosulfonyl)-4-chloro-*N-*(2-méthyl-2,3-dihydro-1*H-*indol-1-yl)benzamide par chromatographie chirale préparative.

### Intermédiaire 9 : 4-(2-Bromoéthyl)benzoate de méthyle

Mettre l'acide 4-(2-bromoéthyl)benzoïque (1,31.10⁻² mol) en suspension dans le méthanol (40 ml). Placer le milieu sous agitation, ajouter de l'acide sulfurique (1 ml) et chauffer à 60 °C. Après environ 10 minutes le milieu est parfaitement limpide. Après 2 heures de chauffage, la réaction est terminée. Laisser le milieu revenir à température ambiante puis le verser sur de l'eau (100 ml). Extraire avec de l'acétate d'éthyle (2 x 100 ml). La phase organique est lavée avec de la saumure, séchée sur sulfate de magnésium, filtrée puis évaporée à sec sous pression réduite.

Le produit du titre est obtenu sous la forme d'une huile incolore utilisée sans purification ultérieure.

### Intermédiaire 10 : 4-(1-Bromoéthyl)benzoate de méthyle

Solubiliser l'acide 4-(1-bromoéthyl)benzoïque (2,18.10⁻³ mol) dans le méthanol (12 ml). Placer le milieu sous agitation puis additionner lentement de l'acide sulfurique (0,5 ml). Après une nuit sous agitation à température ambiante, le milieu réactionnel est versé sur de l'eau (50 ml). Extraire avec du dichlorométhane (3 x 30 ml). La phase organique est lavée avec de la saumure, séchée sur sulfate de magnésium, filtrée puis évaporée à sec sous pression réduite.

Le produit du titre est obtenu sous la forme d'une huile incolore utilisée sans purification ultérieure.

### Exemple 1 : 3-{[({2-Chloro-5-[(2-méthyl-5-nitro-2,3-dihydro-1H-indol-1-yl) carbamoyl]phényl}sulfonyl)amino]méthyl}benzoate de ({[(1Z)-2,2-diéthyl-1-oxidohydrazono]amino}oxy)méthyle

### Stade A : [(2-Chloro-5-{[(2-méthyl-2,3-dihydro-1H-indol-1-yl)amino] carbonyl}phényl)sulfonyl]carbamate de tert-butyle

Mettre en suspension le 3-(aminosulfonyl)-4-chloro-*N-*(2-méthyl-2,3-dihydro-1*H-*indol-1-yl)benzamide (1,37.10⁻² mol) dans le dichlorométhane (125 ml) et placer sous agitation vigoureuse. Ajouter la triéthylamine (1,50.10⁻² mol), la 4-diméthylaminopyridine (1,36.10⁻³ mol) puis une solution de ditertbutyl dicarbonate (1,57.10⁻² mol) dans le dichlorométhane (50 ml). Un dégagement gazeux est observé et rapidement, le milieu réactionnel devient parfaitement limpide. Après 1 heure et 30 minutes, le milieu réactionnel est concentré à sec au rotavapeur. Le résidu obtenu est repris dans l'acétate d'éthyle (50 ml) puis lavé avec de l'eau (200 ml). La phase aqueuse est acidifiée avec une solution 1 N d'acide chlorhydrique dans l'eau puis extraite avec du dichlorométhane. La phase organique est lavée avec de l'eau, de la saumure, séchée sur sulfate de magnésium, filtrée et évaporée à sec sous pression réduite pour conduire au produit du titre sous la forme d'un solide jaune citron utilisé sans purification ultérieure.

### Stade B : 3-({(tert-Butoxycarbonyl)[(2-chloro-5-{[(2-méthyl-2,3-dihydro-1H-indol-1-yl)amino]carbonyl}phényl)sulfonyl]amino}méthyl) benzoate de méthyle

Solubiliser le composé obtenu au Stade A (1,29.10⁻³ mol) dans l'acétonitrile (6 ml), ajouter de la diisopropyléthylamine (1,54.10⁻³ mol) et placer sous agitation. Additionner le méthyl-3-bromométhylbenzoate (1,41.10⁻³ mol) et chauffer le milieu réactionnel à 80 °C. Après 3 heures de chauffage, le milieu est évaporé à sec au rotavapeur. Le brut obtenu est chromatographié sur une colonne de silice en éluant avec un mélange *n-*heptane/acétate d'éthyle 90/10 puis 75/25.

Le produit du titre est obtenu sous la forme d'un solide jaune.

### Stade C : Acide 3-({(tert-butoxycarbonyl)[(2-chloro-5-{[(2-méthyl-2,3-dihydro-1H-indol-1-yl)amino]carbonyl}phényl)sulfonyl]amino} méthyl)benzoïque

Solubiliser le composé obtenu au Stade B (1,14.10⁻³ mol) dans un mélange acétonitrile/eau (40 ml/8 ml). Ajouter de la lithine (1,14.10⁻² mol) et chauffer à 50 °C sous agitation. Après 2 heures de chauffage, le milieu réactionnel est versé sur de l'eau (100 ml). Extraire 3 fois avec de l'acétate d'éthyle (50 ml). La phase organique est ensuite lavée avec de l'eau et de la saumure, séchée sur sulfate de magnésium, filtrée et évaporée à sec sous pression réduite.

Le produit du titre est obtenu après séchage sous une rampe à vide sous la forme d'un solide marron et utilisé sans purification ultérieure.

### Stade D : 3-({(tert-Butoxycarbonyl)[(2-chloro-5-{[(2-méthyl-5-nitro-2,3-dihydro-1H-indol-1-yl)amino]carbonyl}phényl)sulfonyl]amino} méthyl)benzoate de ({[(1Z)-2,2-diéthyl-1-oxidohydrazono]amino} oxy)méthyle

Solubiliser le composé obtenu au Stade C (8,41.10⁻⁴ mol) dans le diméthylformamide (5 ml). Placer sous agitation et sous azote puis ajouter une solution de *N-*[(*Z*)-(chlorométhoxy)-*NNO*-azoxy]-*N-*éthyléthanamine (9,25.10⁻⁴ mol) dans le diméthylformamide (4 ml). Additionner en une fois du carbonate de césium (8,41.10⁻⁴ mol) et laisser le milieu réactionnel sous agitation à température ambiante. Après 2 heures, le milieu est versé sur de l'eau (50 ml). Extraire 3 fois avec de l'acétate d'éthyle (20 ml). La phase organique est lavée avec de la saumure, séchée sur sulfate de magnésium, filtrée et concentrée sous pression réduite.

Le bruit huileux orangé obtenu est chromatographié sur une colonne de silice en éluant avec un mélange *n-*heptane/acétate d'éthyle 65/35 puis 50/50.

Le produit du titre est obtenu sous la forme d'un solide jaune.

### Stade E : 3-{[({2-Chloro-5-[(2-méthyl-5-nitro-2,3-dihydro-1H-indol-1-yl) carbamoyl]phényl}sulfonyl)amino]méthyl}benzoate de ({[(1Z)-2,2-diéthyl-1-oxidohydrazono]amino}oxy)méthyle

Solubiliser le composé obtenu au Stade D (4,38.10⁻⁴ mol) dans le dioxane (25 ml). Placer sous agitation puis additionner de l'acide chlorhydrique concentré à 37 % (3,5 ml) et chauffer à 70 °C. Après 2 heures, le milieu réactionnel est versé sur de l'eau (50 ml). Extraire 3 fois avec de l'acétate d'éthyle (20 ml). La phase organique est lavée avec de l'eau et de la saumure, séchée sur sulfate de magnésium, filtrée et concentrée au rotavapeur. Le brut huileux orangé obtenu est chromatographié sur une colonne de silice en éluant avec un mélange *n-*heptane/acétate d'éthyle 60/40.

Le produit du titre est obtenu sous la forme d'un solide légèrement jaune après séchage sous une rampe à vide à 60 °C.

### Point de fusion : 85-86 °C

| *Microanalyse élémentaire :* | *C* | *H* | *N* | *S* |
|---|---|---|---|---|
| *% théorique :* | *50,47* | *4, 67* | *14, 21* | *4, 65* |
| *% expérimental :* | *51,14* | *5, 04* | *13,19* | *4,55* |

### Exemple 2 : 3-{[({2-Chloro-5-[(2-méthyl-2,3-dihydro-1H-indol-1-yl)carbamoyl] phényl}sulfonyl)amino]méthyl}benzoate de ({(1Z)-2,2-diéthyl-1-oxidohydrazono]amino}oxy)méthyle

### Stade A : 3-({(tert-Butoxycarbonyl)[(2-chloro-5-{[(2-méthyl-2,3-dihydro-1H-indol-1-yl)amino]carbonyl}phényl)sulfonyl]amino}méthyl) benzoate de({[(1Z)-2,2-diéthyl-1-oxidohydrazono]amino}oxy) méthyle

Le produit obtenu au Stade C de l'Exemple 1 est mis en réaction avec le *N-*[(*Z*)-(chlorométhoxy)-*NNO*-azoxy]-*N-*éthyléthanamine selon les conditions opératoires décrites au stade D de l'Exemple 1. Le produit du titre est obtenu sous la forme d'un solide jaune après une chromatographie sur colonne de silice en éluant avec un mélange *n-*heptane/acétate d'éthyle 65/35.

### Stade B : 3-{[({2-Chloro-5-[(2-méthyl-2,3-dihydro-1H-indol-1-yl)carbamoyl] phényl}sulfonyl)amino]méthyl}benzoate de ({[(1Z)-2,2-diéthyl-1-oxidohydrazono]amino}oxy)méthyle

Mode opératoire identique au Stade E de l'Exemple 1 à partir du composé obtenu au Stade A. Le produit du titre est obtenu sous la forme d'un solide blanchâtre après une chromatographie sur colonne de silice en éluant avec un mélange *n-*heptane/acétate d'éthyle 60/40. Il est ensuite séché sous une rampe à vide à 60 °C pendant 24 heures.

### Point de fusion : 66-67 °C

| *Microanalyse élémentaire :* | *C* | *H* | *N* | *S* |
|---|---|---|---|---|
| *% théorique :* | *53,99* | *5,16* | *13, 03* | *4,97* |
| *% expérimental :* | *54,76* | *5*,*50* | *12, 20* | *4,98* |

### Exemple 3 : ({2-Chloro-5-[(2-méthyl-2,3-dihydro-1H-indol-1-yl)carbamoyl]phényl} sulfonyl)[({[(1Z)-2,2-diétbyl-1-oxidohydrazono]amino}oxy)méthyl] carbamate de tert-butyle

Le produit obtenu au Stade A de l'Exemple 1 (1,00.10⁻³ mol) est solubilisé dans de l'acétonitrile (5 ml). La solution est placée sous agitation. Ajouter de la diisopropyl éthylamine (1,20.10⁻³ mol) puis une solution de **N-**[(*Z*)-(chlorométhoxy)-*NNO-*azoxy]-*N-*éthyléthanamine (1,10.10⁻³ mol) dans l'acétonitrile (2 ml). Chauffer le milieu réactionnel à 70 °C pendant 2 heures et 30 minutes. Verser le milieu réactionnel sur de l'eau (20 ml) et extraire 3 fois avec de l'acétate d'éthyle (15 ml). La phase organique est lavée avec de l'eau et de la saumure, séchée sur sulfate de magnésium, filtrée et concentrée à sec au rotavapeur. Le brut huileux orangé obtenu est chromatographié sur une colonne de silice en éluant avec un mélange *n-*heptane/acétate d'éthyle 80/20. Un solide jaunâtre est récupéré. Le produit du titre est obtenu sous la forme d'un solide orangé après une gel-filtration sur Séphadex LH-20 en éluant avec un mélange acétone/dichlorométhane 1/1.

### Point de fusion : 80-81 °C

| *Microanalyse élémentaire :* | *C* | *H* | *N* | *S* |
|---|---|---|---|---|
| *% théorique :* | *51,10* | *5,77* | *13,75* | *5,25* |
| *% expérimental :* | *51,36* | *5,90* | *13,16* | *5,34* |

### Exemple 4 : N-(tert-Butoxycarbonyl)-N-({2-chloro-5-[(2-méthyl-2,3-dihydro-1H-indol-1-yl)carbamoyl]phényl}sulfonyl)glycinate de ({[(1Z)-2,2-diéthyl-1-oxidohydrazono]amino}oxy)méthyle

### Stade A : N-(tert-Butoxycarbonyl)-N-[(2-chloro-5-{[(2-méthyl-2,3-dihydro-1H-indol-1-yl)amino]carbonyl}phényl)sulfonyl]glycinate de benzyle

Le composé obtenu au Stade A de l'Exemple 1 est mis en réaction avec du bromoacétate de benzyle selon les conditions opératoires décrites au Stade B de l'Exemple 1. Le brut huileux jaunâtre obtenu est chromatographié sur colonne de silice en éluant avec un mélange *n-*heptane/acétate d'éthyle 80/20.

Le produit du titre est obtenu sous la forme d'un solide légèrement jaune.

### Stade B : N-(tert-Butoxycarbonyl)-N-[(2-chloro-5-{[(2-méthyl-2,3-dihydro-1H-indol-1-yl)amino]carbonyl}phényl)sulfonyl]glycine

Le composé obtenu au Stade A (1,34.10⁻³ mol) est solubilisé dans l'acétate d'éthyle (50 ml). Ajouter le catalyseur au palladium sur charbon (10 %, 82 mg) puis placer le milieu réactionnel à température ambiante sous hydrogène à pression atmosphérique. Après 24 heures, le milieu réactionnel est filtré sur célite puis le filtrat est évaporé à sec sous pression réduite. Le résidu huileux orangé obtenu est chromatographié sur une colonne de silice en éluant avec du dichlorométhane puis un mélange dichlorométhane/méthanol 98/2, 95/5 et 90/10.

Le produit du titre est obtenu sous la forme d'un solide jaunâtre.

### Stade C : N-(tert-Butoxycarbonyl)-N-({2-chloro-5-[(2-méthyl-2,3-dihydro-1H-indol-1-yl)carbamoyl]phényl}sulfonyl)glycinate de ({[(1Z)-2,2-diéthyl-1-oxidohydrazono]amino}oxy)méthyle

Solubiliser le produit obtenu au Stade B (1,01.10⁻³ mol) dans le diméthylformamide (5 ml). Placer sous agitation et ajouter une solution de *N-*[(*Z*)-(chlorométhoxy)-*NNO*-azoxy]-*N-*éthyléthanamine (2,23.10⁻³ mol) dans le diméthylformamide (3 ml). Additionner ensuite en une fois du carbonate de césium (1,01.10⁻³ mol). Après 2 heures, le milieu réactionnel est versé sur de l'eau (30 ml). Extraire 3 fois avec de l'acétate d'éthyle (20 ml). La phase organique est lavée avec de l'eau et de la saumure, séchée sur sulfate de magnésium, filtrée et concentrée sous pression réduite. Le résidu obtenu est chromatographié sur une colonne de silice en éluant avec un mélange *n-*heptane/acétate d'éthyle 80/20 puis 70/30.

Le produit du titre est obtenu sous la forme d'un solide légèrement jaune.

### Point de fusion : 76-77 °C

| *Microanalyse élémentaire :* | *C* | *H* | *N* | *S* |
|---|---|---|---|---|
| *% théorique :* | *50, 26* | *5,57* | *12,56* | *4,79* |
| *% expérimental :* | *50,34* | *5,74* | *12,36* | *4,65* |

### Exemple 5 : N-({2-Chloro-5-[(2-méthyl-2,3-dihydro-1H-indol-1-yl)carbamoyl] phényl}sulfonyl)glycinate de ({[(1Z)-2,2-diéthyl-1-oxidohydrazono] amino}oxy)méthyle

Le composé obtenu au Stade C de l'Exemple 4 (4,06.10⁻⁴ mol) est solubilisé dans du dichlorométhane (12 ml). La solution est refroidie à 0 °C et placée sous agitation. Additionner de l'acide trifluoroacétique (4,06.10⁻³ mol). Après 1 heure, le milieu réactionnel est placé à température ambiante. Après 3 jours, le milieu est concentré à sec au rotavapeur. Le brut huileux récupéré est chromatographié sur une colonne de silice en éluant avec un mélange *n-*heptane/acétate d'éthyle 60/40 puis 50/50.

Le produit du titre est obtenu sous la forme d'un solide jaune après une gel-filtration sur Séphadex LH-20 en éluant avec un mélange acétone/dichlorométhane 1/1.

### Point de fusion : 68-69 °C

| *Microanalyse élémentaire :* | *C* | *H* | *N* | *S* |
|---|---|---|---|---|
| *% théorique :* | *48,55* | *5,14* | *14,77* | *5,64* |
| *% expérimental :* | *48,16* | *5*,*24* | *14,17* | *5,46* |

### Exemple 6 : Pentanedioate de [(tert-butoxycarbonyl)({2-chloro-5-[(2-méthyl-2,3-dihydro-1H-indol-1-yl)carbamoyl]phényl}sulfonyl)amino]méthyle et de ({[(1Z)-2,2-diéthyl-1-oxidohydrazono]amino}oxy)méthyle

### Stade A : Pentanedioate de benzyle et de [(tert-butoxycarbonyl)({2-chloro-5-[(2-méthyl-2,3-dihydro-1H-indol-1-yl)carbamoyl]phényl}sulfonyl) amino]méthyle

Solubiliser le composé obtenu au Stade A de l'Exemple 1 (2,15.10⁻³ mol) dans l'acétonitrile (10 ml) et placer la solution sous agitation. Ajouter la diisopropyléthylamine (2,57.10⁻³ mol) puis une solution de l'intermédiaire 3 (2,36.10⁻³ mol) dans l'acétonitrile (10 ml). Lors de l'addition le milieu réactionnel devient rougeâtre puis se décolore rapidement. Chauffer le milieu à 60 °C. Après 1 heure et 30 minutes, le milieu réactionnel est évaporé à sec sous pression réduite. Le résidu solide jaune obtenu est chromatographié sur colonne de silice en éluant avec un mélange *n-*heptane/acétate d'éthyle 80/20 puis 70/30.

Le produit du titre est obtenu sous la forme d'un solide jaune.

### Stade B : Acide 5-{[(tert-butoxycarbonyl)({2-chloro-5-[(2-méthyl-2,3-dihydro-1H-indol-1-yl)carbamoyl]phényl}sulfonyl)amino] méthoxy}-5-oxopentanoïque

Solubiliser le composé obtenu au Stade A (1,45.10⁻³ mol) dans de l'acétate d'éthyle (100 ml). Ajouter le catalyseur au palladium sur charbon (10 %, 100 mg) puis placer le milieu réactionnel à température ambiante sous hydrogène à pression atmosphérique. Après 36 heures, le milieu réactionnel est filtré sur célite et du catalyseur au palladium sur charbon neuf est à nouveau ajouté (10 %, 100 mg). Le milieu est replacé sous hydrogène dans les mêmes conditions que celles décrites ci-dessus pendant 24 heures supplémentaires. La réaction est alors terminée, le milieu est filtré sur célite puis le filtrat est évaporé à sec sous pression réduite. Le résidu obtenu est chromatographié sur colonne de silice en éluant avec un mélange dichlorométhane/méthanol 98/2 puis 97/3.

Le produit du titre est obtenu sous la forme d'un solide couleur crème.

### Stade C : Pentanedioate de [(tert-butoxycarbonyl)({2-chloro-5-[(2-méthyl-2,3-dihydro-1H-indol-1-yl)carbamoyl]phényl}sulfonyl)amino] méthyle et de ({[(1Z)-2,2-diéthyl-1-oxidohydrazono]amino}oxy) méthyle

Solubiliser le composé obtenu au stade B (9,23.10⁻⁴ mol) dans le diméthylformamide (10 ml). Placer sous agitation et sous azote et ajouter une solution de *N-*[(*Z*)-(chlorométhoxy)-*NNO*-azoxy]-*N-*éthyléthanamine (1,11.10⁻³ mol) dans le diméthylformamide (3 ml). Additionner ensuite en une fois du carbonate de césium (9,23.10⁻⁴ mol). Après 1 heure et 30 minutes, le milieu réactionnel est versé sur de l'eau (100 ml). Extraire 3 fois avec de l'acétate d'éthyle (75 ml). La phase organique est lavée avec de l'eau puis de la saumure. Elle est ensuite séchée sur sulfate de magnésium, filtrée puis évaporée à sec sous pression réduite. Le résidu huileux orangé obtenu est purifié par chromatographie sur colonne de silice en éluant avec un mélange *n-*heptane/acétate d'éthyle 70/30 puis 60/40. Une deuxième chromatographie en phase inverse sur colonne de Lichroprep RP-18 est nécessaire. Les conditions d'élution sont les suivantes : (éluant A : 1000 ml H₂O/25 ml CH₃CN ; éluant B : 25 ml H₂O/1000 ml CH₃CN) 10 % de B pendant 15 minutes, de 10 à 75 % de B en 60 minutes, de 75 à 100 % de B en 20 minutes.

Le produit du titre est obtenu sous la forme d'une meringue légèrement jaunâtre.

| *Microanalyse élémentaire :* | *C* | *H* | *N* | *S* |
|---|---|---|---|---|
| *% théorique :* | *50,89* | *5,74* | *11,13* | *4, 25* |
| *% expérimental :* | *50,83* | *5,74* | *11,16* | *3,99* |

### Exemple 7 : Butanedioate de [(tert-butoxycarbonyl)({2-chloro-5-[(2-méthyl-2,3-dihydro-1N indol-1-yl)carbamoyl]phényl}sulfonyl)amino]méthyle et de ({[(1Z)-2,2-diéthyl-1-oxidohydrazono]amino}oxy)méthyle

### Stade A : Butanedioate de benzyle et de [(tert-butoxycarbonyl)({2-chloro-5-[(2-méthyl-2,3-dihydro-1H-indol-1-yl)carbamoyl]phényl}sulfonyl) amino]méthyle

Solubiliser le composé obtenu au Stade A de l'Exemple 1 (1,50.10⁻³ mol) dans l'acétonitrile (7 ml) et placer la solution sous agitation. Ajouter la diisopropyléthylamine (1,80.10⁻³ mol) puis une solution de l'intermédiaire 6 (1,65.10⁻³ mol) dans l'acétonitrile (7 ml). Lors de l'addition le milieu réactionnel devient rougeâtre puis se décolore rapidement. Chauffer le milieu à 55 °C. Après environ 30 minutes, le milieu réactionnel est évaporé à sec sous pression réduite. Le résidu solide jaune citron obtenu est chromatographié sur colonne de silice en éluant avec un mélange *n-*heptane/acétate d'éthyle 80/20, 70/30 puis 60/40.

Le produit du titre est obtenu sous la forme d'une meringue jaune.

### Stade B : Acide 4-{[(tert-butoxycarbonyl)({2-chloro-5-[(2-méthyl-2,3-dihydro-1H-indol-1-yl)carbamoyl]phényl}sulfonyl)amino] méthoxy}-4-oxobutanoïque

Solubiliser le composé obtenu au Stade A (1,08.10⁻³ mol) dans de l'acétate d'éthyle (75 ml). Ajouter le catalyseur au palladium sur charbon (10 %, 75 mg) puis placer le milieu réactionnel à température ambiante sous hydrogène à pression atmosphérique. Après 36 heures, le milieu réactionnel est filtré sur célite et du catalyseur au palladium sur charbon neuf est à nouveau ajouté (10 %, 75 mg). Le milieu est replacé sous hydrogène dans les mêmes conditions que celles décrites ci-dessus pendant 24 heures supplémentaires. La réaction est alors terminée, le milieu est filtré sur célite puis le filtrat est évaporé à sec sous pression réduite. Le résidu obtenu est chromatographié sur colonne de silice en éluant avec un mélange dichlorométhane/méthanol 98/2.

Le produit du titre est obtenu sous la forme d'un solide couleur crème.

### Stade C : Butanedioate de [(tert-butoxycarbonyl)({2-chloro-5-[(2-méthyl-2,3-dihydro-1H-indol-1-yl)carbamoyl]phényl}sulfonyl)amino] méthyle et de ({[(1Z)-2,2-diéthyl-1-oxidohydrazono]amino}oxy) méthyle

Solubiliser le composé obtenu au Stade B (8,30.10⁻⁴ mol) dans le diméthylformamide (9 ml). Placer sous agitation et sous azote et ajouter une solution de *N-*[(*Z*)-(chlorométhoxy)-*NNO*-azoxy]-*N-*éthyléthanamine (9,96.10⁻⁴ mol) dans le diméthylformamide (3 ml). Additionner ensuite en une fois du carbonate de césium (8,30.10⁻⁴ mol). Après 1 heure, le milieu réactionnel est versé sur de l'eau (100 ml). Extraire 3 fois avec de l'acétate d'éthyle (75 ml). La phase organique est lavée avec de l'eau puis de la saumure. Elle est ensuite séchée sur sulfate de magnésium, filtrée puis évaporée à sec sous pression réduite. Le résidu huileux orangé obtenu est purifié par chromatographie sur colonne de silice en éluant avec un mélange *n-*heptane/acétate d'éthyle 70/30 puis 60/40. Une deuxième chromatographie en phase inverse sur colonne de Lichroprep RP-18 (60x400mm) est nécessaire. Les conditions d'élution sont les suivantes : (éluant A : 1000 ml H₂O/25 ml CH₃CN ; éluant B : 25 ml H₂O/1000 ml CH₃CN) A/B : 40/60 ; débit : 12 ml/min.

Le produit du titre est obtenu sous la forme d'une meringue orangée.

| *Microanalyse élémentaire :* | *C* | *H* | *N* | *S* |
|---|---|---|---|---|
| *% théorique :* | *50,23* | *5,58* | *11,34* | *4,33* |
| *% expérimental :* | *50,51* | *5, 67* | *10,91* | *4,48* |

### Exemple 8 : 4-{[({2-Chloro-5-[(2-méthyl-2,3-dihydro-1H-indol-1-yl)carbamoyl] phényl}sulfonyl)amino]méthyl}benzoate de ({[(1Z-2,2-diéthyl-1-oxidohydrazono]amino}oxy)méthyle

### Stade A : 4-{[(tert-Butoxycarbonyl)({2-chloro-5-[(2-méthyl-2,3-dihydro-1H-indol-1-yl)carbamoyl]phényl}sulfonyl)amino]méthyl}benzoate de méthyle

Solubiliser le composé obtenu au Stade A de l'Exemple 1 (1,72.10⁻³ mol) dans l'acétonitrile (8 ml) et placer la solution sous agitation. Ajouter la diisopropyléthylamine (2,06.10⁻³ mol) puis après 10 minutes d'agitation, le méthyl 4-bromométhylbenzoate (2,57.10⁻³ mol). Chauffer le milieu à 60 °C. Après 2 heures et 30 minutes, le milieu réactionnel est versé sur de l'eau (30 ml). Extraire 3 fois avec de l'acétate d'éthyle (20 ml). La phase organique est lavée avec de la saumure, séchée sur sulfate de magnésium, filtrée et évaporée à sec sous pression réduite. Le résidu huileux orangé obtenu est chromatographié sur colonne de silice en éluant avec un mélange *n-*heptane/acétate d'éthyle 90/10 à 75/25.

Le produit du titre est obtenu sous la forme d'une meringue jaune.

### Stade B : Acide 4-{[(tert-butoxycarbonyl)({2-chloro-5-[(2-méthyl-2,3-dihydro-1H-indol-1-yl)carbamoyl]phényl}sulfonyl)amino]méthyl} benzoïque

Solubiliser le composé obtenu au Stade A (1,30.10⁻³ mol) dans un mélange acétonitrile (45 ml) et eau (9 ml). Ajouter de la lithine (1,30.10⁻² mol) et chauffer à 50 °C sous agitation. Après 5 heures de chauffage, le milieu réactionnel marron foncé est versé sur de l'eau (100 ml). Extraire 3 fois avec de l'acétate d'éthyle (50 ml). La phase organique est ensuite lavée avec de l'eau et de la saumure, séchée sur sulfate de magnésium, filtrée et évaporée à sec sous pression réduite.

Le produit du titre est obtenu après séchage sous une rampe à vide sous la forme d'une meringue marron utilisée sans purification ultérieure.

### Stade C : 4-{[(tert-Butoxycarbonyl)({2-chloro-5-[(2-méthyl-2,3-dihydro-1H-indol-1-yl)carbamoyl]phényl}sulfonyl)amino]méthyl}benzoate de ({[(1Z)-2,2-diéthyl-1-oxidohydrazono]amino}oxy)méthyle

Solubiliser le composé obtenu au Stade B (8,96.10⁻⁴ mol) dans le diméthylformamide (8 ml). Placer sous agitation et sous azote puis ajouter une solution de *N-*[(*Z*)-(chlorométhoxy)-*NNO*-azoxy]-*N-*éthyléthanamine (1,17.10⁻³ mol) dans le diméthylformamide (1 ml). Additionner en une fois du carbonate de césium (9,41.10⁻⁴ mol) et laisser le milieu réactionnel sous agitation à température ambiante. Après 2 heures, le milieu est versé sur de l'eau (50 ml). Extraire 3 fois avec de l'acétate d'éthyle (20 ml). La phase organique est lavée avec de la saumure, séchée sur sulfate de magnésium, filtrée et concentrée sous pression réduite.

Le brut huileux orangé obtenu est chromatographié sur une colonne de silice en éluant avec un mélange *n-*heptane/acétate d'éthyle 65/35.

Le produit du titre est obtenu sous la forme d'un solide jaune.

### Stade D : 4-{[({2-Chloro-5-[(2-méthyl-2,3-dihydro-1H-indol-1-yl)carbamoyl] phényl}sulfonyl)amino]méthyl}benzoate de ({[(1Z)-2,2-diéthyl-1-oxidohydrazono]amino}oxy)méthyle

Solubiliser le composé obtenu au Stade C (4,74.10⁻⁴ mol) dans le 1,4-dioxane (5 ml). Placer la solution sous agitation à température ambiante puis ajouter une solution d'acide chlorhydrique 4 N dans le 1,4-dioxane (10 ml). Le milieu réactionnel initialement jaunâtre devient de plus en plus foncé jusqu'à être quasiment noir. Après 2 jours d'agitation à température ambiante, le milieu réactionnel est évaporé à sec sous pression réduite. Le résidu noir obtenu est chromatographié sur une colonne de silice en éluant avec un mélange *n-*heptane/acétate d'éthyle 50/50.

Le produit du titre est obtenu après recristallisation dans le *n-*heptane sous la forme d'un solide blanc.

### Point de fusion : 96-97 °C

| *Microanalyse élémentaire :* | *C* | *H* | *N* | *S* |
|---|---|---|---|---|
| *% théorique :* | *53,99* | *5,16* | *13,03* | *4,97* |
| *% expérimental :* | *54,24* | *5,27* | *12,65* | *4,60* |

### Exemple 9 : 2-{[(tert-Butoxycarbonyl)({2-chloro-5-[(2-méthyl-2,3-dihydro-1H-indol-1-yl)carbamoyl]phényl}sulfonyl)amino]méthyl}benzoate de ({[(1Z)-2,2-diéthyl-1-oxidohydrazono]amino}oxy)méthyle

### Stade A : 2-{[(tert-Butoxycarbonyl)({2-chloro-5-[(2-méthyl-2,3-dihydro-1H-indol-1-yl)carbamoyl]phényl}sulfonyl)amino]méthyl}benzoate de méthyle

Solubiliser le composé obtenu au Stade A de l'Exemple 1 (1,72.10⁻³ mol) dans l'acétonitrile (8 ml) et placer la solution sous agitation. Ajouter la diisopropyléthylamine (2,06.10⁻³ mol) puis après 10 minutes d'agitation, l'intermédiaire 7 (2,62.10⁻³ mol). Chauffer le milieu à 60 °C. Après 5 heures, le milieu réactionnel est versé sur de l'eau (30 ml). Extraire 3 fois avec de l'acétate d'éthyle (20 ml). La phase organique est lavée avec de la saumure, séchée sur sulfate de magnésium, filtrée et évaporée à sec sous pression réduite. Le résidu huileux orangé-marron obtenu est chromatographié sur colonne de silice en éluant avec un mélange *n-*heptane/acétate d'éthyle 90/10 à 65/35.

Le produit du titre est obtenu sous la forme d'une meringue jaune.

### Stade B : Acide 2-{[(tert-butoxycarbonyl)({2-chloro-5-[(2-méthyl-2,3-dihydro-1H-indol-1-yl)carbamoyl]phényl}sulfonyl)amino]méthyl} benzoïque

Solubiliser le composé obtenu au Stade A (1,26.10⁻³ mol) dans un mélange acétonitrile (45 ml) et eau (9 ml). Ajouter de la lithine (1,26.10⁻² mol) et chauffer à 50 °C sous agitation. Après 5 heures et 30 minutes de chauffage, le milieu réactionnel marron foncé est versé sur de l'eau (100 ml). Extraire 3 fois avec de l'acétate d'éthyle (50 ml). La phase organique est ensuite lavée avec de l'eau et de la saumure, séchée sur sulfate de magnésium, filtrée et évaporée à sec sous pression réduite.

Le produit du titre est obtenu après séchage sous une rampe à vide sous la forme d'une meringue marron utilisée sans purification ultérieure.

### Stade C : 2-{[(tert-Butoxycarbonyl)({2-chloro-5-[(2-méthyl-2,3-dihydro-1H-indol-1-yl)carbamoyl]phényl}sulfonyl)amino]méthyl}benzoate de ({[(1Z)-2,2-diéthyl-1-oxidohydrazono]amino}oxy)méthyle

Solubiliser le composé obtenu au Stade B (8,25.10⁻⁴ mol) dans le diméthylformamide (8 ml). Placer sous agitation et sous azote puis ajouter une solution de *N-*[(*Z*)-(chlorométhoxy)-*NNO*-azoxy]-*N-*éthyléthanamine (1,07.10⁻³ mol) dans le diméthylformamide (2 ml). Additionner en une fois du carbonate de césium (8,66.10⁻⁴ mol) et laisser le milieu réactionnel sous agitation à température ambiante. Après 3 heures, le milieu est versé sur de l'eau (50 ml). Extraire 3 fois avec de l'acétate d'éthyle (20 ml). La phase organique est lavée avec de la saumure, séchée sur sulfate de magnésium, filtrée et concentrée sous pression réduite.

Le brut huileux marron obtenu est chromatographié sur une colonne de silice en éluant avec un mélange *n-*heptane/acétate d'éthyle 70/30. Une deuxième chromatographie en phase inverse sur colonne de Lichroprep RP-18 est nécessaire. Les conditions d'élution sont les suivantes : (éluant A : 1000 ml H₂O/25 ml CH₃CN ; éluant B : 25 ml H₂O/1000 ml CH₃CN) 20 % de B pendant 15 minutes, de 20 à 83 % de B en 60 minutes, de 83 à 100 % de B en 15 minutes.

Le produit du titre est obtenu sous la forme d'un solide jaune après recristallisation dans un mélange *n-*heptane/acétate d'éthyle.

### Point de fusion : 79-81 °C

| *Microanalyse élémentaire :* | *C* | *H* | *N* | *S* |
|---|---|---|---|---|
| *% théorique :* | *54,80* | *5,54* | *11, 28* | *4,30* |
| *% expérimental:* | *54,57* | *5,65* | *10,82* | *3,88* |

### Exemple 10 : 4-({[(2-Chloro-5-{[(2R)-2-méthyl-2,3-dihydro-1H-indol-1-yl]carbamoyl} phényl)sulfonyl]amino}méthyl)benzoate de ({[(1Z)-2,2-diéthyl-1-oxidohydrazono]amino}oxy)méthyle

### Stade A : [(2-Chloro-5-{[(2R)-2-méthyl-2,3-dihydro-1H-indol-1-yl] carbamoyl}phényl)sulfonyl]carbamate de tert-butyle

Mettre en suspension l'intermédiaire 8 (6,0.10⁻³ mol) dans le dichlorométhane (55 ml) et placer sous agitation vigoureuse. Ajouter de la triéthylamine (6,60.10⁻³ mol), de la 4-diméthylaminopyridine (6,0.10⁻⁴ mol) puis une solution de ditertbutyl dicarbonate (6,90.10⁻³ mol) dans le dichlorométhane (20 ml). Un dégagement gazeux est observé et rapidement, le milieu réactionnel devient parfaitement limpide. Après 1 heure et 30 minutes, le milieu réactionnel est versé sur de l'eau (100 ml). Extraire 3 fois avec du dichlorométhane (50 ml). La phase organique est ensuite lavée avec de l'eau, de la saumure, séchée sur sulfate de magnésium, filtrée et évaporée à sec sous pression réduite. Le produit du titre est obtenu sous la forme d'un solide jaune citron utilisé sans purification ultérieure.

### Stade B : 4-({(tert-Butoxycarbonyl)[(2-chloro-5-{[(2R)-2-méthyl-2,3-dihydro-1H-indol-1-yl]carbamoyl}phényl)sulfonyl]amino}méthyl) benzoate de méthyle

Solubiliser le composé obtenu au Stade A (7,57.10⁻³ mol) dans l'acétonitrile (35 ml) et placer la solution sous agitation. Ajouter la diisopropyléthylamine (9,09.10⁻³ mol) puis après 10 minutes d'agitation, le méthyl 4-bromométhylbenzoate (1,15.10⁻⁴ mol). Chauffer le milieu à 60 °C. Après une nuit, le milieu réactionnel est versé sur de l'eau (100 ml). Extraire 3 fois avec de l'acétate d'éthyle (50 ml). La phase organique est lavée avec de la saumure, séchée sur sulfate de magnésium, filtrée et évaporée à sec sous pression réduite. Le résidu obtenu est trituré dans un mélange *n-*heptane/acétate d'éthyle entraînant la précipitation d'un solide récupéré par filtration.

Le produit du titre est obtenu sous la forme d'un solide blanc.

### Stade C : Acide 4-({(tert-butoxycarbonyl)[(2-chloro-5-{(2R)-2-méthyl-2,3-dihydro-1H-indol-1-yl]carbamoyl}phényl)sulfonyl]amino}méthyl) benzoïque

Solubiliser le composé obtenu au Stade B (5,03.10⁻³ mol) dans un mélange acétonitrile (175 ml) et eau (35 ml). Ajouter de la lithine (5,03.10⁻² mol) et chauffer à 50 °C sous agitation. Après 3 heures de chauffage, le milieu réactionnel marron foncé est versé sur de l'eau (200 ml). Extraire 3 fois avec de l'acétate d'éthyle (150 ml). La phase organique est ensuite lavée avec de l'eau et de la saumure, séchée sur sulfate de magnésium, filtrée et évaporée à sec sous pression réduite. Le résidu huileux obtenu est trituré dans un mélange *n-*heptane/acétate d'éthyle entraînant la précipitation d'un solide récupéré par filtration.

Le produit du titre est obtenu sous la forme d'un solide couleur crème.

### Stade D : 4-({(tert-Butoxycarbonyl)[(2-chloro-5-{[(2R)-2-méthyl-2,3-dihydro-1H-indol-1-yl]carbamoyl}phényl)sulfonyl]amino}méthyl) benzoate de ({[(1Z)-2,2-diéthyl-1-oxidohydrazono]amino}oxy) méthyle

Solubiliser le composé obtenu au Stade C (2,90.10⁻³ mol) dans le diméthylformamide (30 ml). Placer sous agitation et sous azote puis ajouter une solution de *N-*[(*Z*)-(chlorométhoxy)-*NNO*-azoxyl-*N-*éthyléthanamine (4,88.10⁻³ mol) dans le diméthylformamide (3 ml). Additionner en une fois du carbonate de césium (3,05.10⁻³ mol) et laisser le milieu réactionnel sous agitation à température ambiante. Après 3 heures et 30 minutes, le milieu est versé sur de l'eau (200 ml). Extraire 3 fois avec de l'acétate d'éthyle (150 ml). La phase organique est lavée avec de la saumure, séchée sur sulfate de magnésium, filtrée et concentrée sous pression réduite.

Le brut huileux orangé obtenu est chromatographié sur une colonne de silice en éluant avec un mélange *n-*heptane/acétate d'éthyle 65/35.

Le produit du titre est obtenu sous la forme d'un solide jaune.

### Stade E : 4-({[(2-Chloro-5-{[(2R)-2-méthyl-2,3-dihydro-1H-indol-1-yl] carbamoyl}phényl)sulfonyl]amino}méthyl)benzoate de ({[(1Z)-2,2-diéthyl-1-oxidohydrazono]amino}oxy)méthyle

Solubiliser le composé obtenu au Stade D (1,47.10⁻³ mol) dans une solution d'acide chlorhydrique 4 N dans le 1,4-dioxane (20 ml) et placer sous agitation. Le milieu réactionnel initialement jaunâtre devient de plus en plus foncé jusqu'à être quasiment noir. Après 18 heures d'agitation à température ambiante, le milieu réactionnel est évaporé à sec sous pression réduite. Le résidu noir obtenu est chromatographié sur une colonne de silice en éluant avec un mélange *n-*heptane/acétate d'éthyle 50/50.

Le produit du titre est obtenu après recristallisation dans le *n-*heptane sous la forme d'un solide blanc.

### Point de fusion : 78-79 °C

| *Microanalyse élémentaire :* | *C* | *H* | *N* | *S* |
|---|---|---|---|---|
| *% théorique :* | *53,99* | *5,16* | *13, 03* | *4,97* |
| *% expérimental :* | *53,77* | *4,94* | *12,90* | *5, 06* |

### Exemple 11 : ({2-Chloro-5-[(2-méthyl-2,3-dihydro-1H-indol-1-yl)carbamoyl]phényl} sulfonyl)amidophosphate d'hydrogène et de ({[(1Z)-2,2-diéthyl-1-oxidohydrazono]amino}oxy)méthyle

### Stade A : ({2-Chloro-5-[(2-méthyl-2,3-dihydro-1H-indol-1-yl)carbamoyl] phényl}sulfonyl)amidophosphate de diéthyle

Dans un tricol surmonté de deux ampoules à addition, solubiliser le 3-(aminosulfonyl)-4-chloro-*N-*(2-méthyl-2,3-dihydro-1*H-*indol-1-yl)benzamide (1,37.10⁻² mol) dans une solution aqueuse 1 N de soude (25 ml). La solution jaune citron obtenue est placée sous agitation à température ambiante et sous azote. Additionner goutte à goutte une solution de diéthylchlorophosphate (9,56.10⁻² mol) dans le tétrahydrofurane (50 ml) et simultanément une solution aqueuse 3 N de soude (45 ml) de manière à maintenir le milieu réactionnel parfaitement homogène. L'addition terminée, le milieu est laissée 2 heures à température ambiante. Verser le milieu réactionnel sur de l'eau (200 ml) et extraire 3 fois avec de l'acétate d'éthyle. La phase aqueuse est acidifiée à pH 4 puis extraite à nouveau avec de l'acétate d'éthyle. Les phases organiques sont rassemblées, séchées sur sulfate de magnésium, filtrées et évaporées à sec sous pression réduite.

Le produit du titre est obtenu sous la forme d'une huile orangée utilisée sans purification ultérieure.

### Stade B : Acide ({2-Chloro-5-[(2-méthyl-2,3-dihydro-1H-indol-1-yl) carbamoyl]phényl}sulfonyl)phosphoramidique

Solubiliser le composé obtenu au Stade A (1,22.10⁻² mol) dans le dichlorométhane (130 ml). Placer la solution sous agitation à 0 °C et sous azote. Additionner ensuite goutte à goutte l'iodure de triméthylsilyle (5,41.10⁻² mol). L'addition terminée, laisser revenir à température ambiante sous agitation. Après 18 heures, le milieu réactionnel est évaporé à sec sous pression réduite. Reprendre le résidu marron dans un mélange acétone/eau 25 ml/l ml, laisser sous agitation pendant 15 minutes puis évaporer à sec à nouveau. Le résidu marron obtenu sous la forme d'une meringue est purifié par chromatographie en phase inverse sur colonne de Lichroprep RP-18. Les conditions d'élution sont les suivantes: (éluant A: 1000 ml H₂O/25 ml CH₃CN ; éluant B: 25 ml H₂O/1000 ml CH₃CN) 100 % de A ; 95/5 puis 90/10 A/B.

Les fractions contenant le produit attendu sont lyophilisées.

Le produit du titre est obtenu sous la forme d'un solide floconneux jaune.

### Stade C : ({2-Chloro-5-[(2-méthyl-2,3-dihydro-1H-indol-1-yl) carbamoyl] phényl}sulfonyl)amidophosphate d'hydrogène et de ({[(1Z)-2,2-diéthyl-1-oxidohydrazono]amino}oxy)méthyle

Solubiliser le composé obtenu au Stade B (3,81.10⁻³ mol) dans la N,N'-diméthyl-N,N'-propylurée (25 ml). Ajouter de la triéthylamine (8,88.10⁻³ mol) puis laisser sous agitation à température ambiante pendant 10 minutes. Ajouter ensuite la *N-*[(*Z*)-(chlorométhoxy)-*NNO*-azoxy]-*N-*éthyléthanamine (15,25.10⁻³ mol), l'iodure de sodium (7,62.10⁻³ mol) et chauffer le milieu réactionnel à 50 °C. Après une nuit, le milieu réactionnel est versé sur de l'éther diéthylique (200 ml). Filtrer les insolubles qui sont repris dans l'acétonitrile. Le solide qui reste en suspension est filtré et éliminé et le filtrat est évaporé à sec sous pression réduite.

Le résidu obtenu est purifié par 3 chromatographies successives en phase inverse sur une colonne de Lichroprep RP-18. Les conditions d'élution sont les suivantes: (éluant A: 1000 ml H₂O/25 ml CH₃CN ; éluant B : 25 ml H₂O/1000 ml CH₃CN) 5 % de B pendant 10 minutes, de 5 à 75 % de B en 60 minutes, de 75 à 100 % de B en 10 minutes. Les fractions contenant le produit attendu sont lyophilisées.

Le produit du titre est obtenu sous la forme d'un solide floconneux jaune.

| *Microanalyse élémentaire :* | *C* | *H* | *N* | S | *Cl* |
|---|---|---|---|---|---|
| *% théorique :* | *42, 68* | *4,78* | *14, 22* | *5,43* | *6, 00* |
| *% expérimental :* | *42,58* | *5,24* | *13, 20* | *5,13* | *5, 62* |

### Exemple 12 : (4-{[({2-Chloro-5-[(2-méthyl-2,3-dihydro-1H-indol-1-yl)carbamoyl] phényl}sulfonyl)amino]méthyl}phényl)acétate de ({[(1Z)-2,2-diéthyl-1-oxidohydrazono]amino}oxy)méthyle

### Stade A : Acide (4-{[(tert-butoxycarbonyl)({2-chloro-5-[(2-méthyl-2,3-dihydro-1H-indol-1-yl)carbamoyl]phényl}sulfonyl)amino]méthyl} phényl)acétique

Solubiliser le composé obtenu au Stade A de l'Exemple 1 (3,22.10⁻³ mol) dans l'acétonitrile (15 ml) et placer la solution sous agitation. Ajouter la diisopropyléthylamine (3,86.10⁻³ mol) puis l'acide 4-bromométhyl-phénylacétique (7,08.10⁻³ mol). Chauffer le milieu à 65 °C. Après 3 heures, le milieu réactionnel est évaporé à sec sous pression réduite. Le résidu huileux orangé obtenu est chromatographié sur colonne de silice en éluant avec un mélange *n-*heptane/acétate d'éthyle 60/40 puis 50/50.

Le produit du titre est obtenu sous la forme d'un solide blanchâtre.

### Stade B : (4-{[(tert-Butoxycarbonyl)({2-chloro-5-[(2-méthyl-2,3-dihydro-1H-indol-1-yl)carbamoyl]phényl}sulfonyl)amino]méthyl}phényl) acétate de ({[(1Z)-2,2-diéthyl-1-oxidohydrazono]amino}oxy) méthyle

Solubiliser le composé obtenu au Stade A (2,03.10⁻³ mol) dans le diméthylformamide (10 ml). Placer sous agitation et sous azote puis ajouter une solution de *N-*[(*Z*)-(chlorométhoxy)-*NNO*-azoxy]-*N-*éthyléthanamine (2,64.10⁻³ mol) dans le diméthylformamide (2 ml). Additionner en une fois du carbonate de césium (2,13.10⁻³ mol) et laisser le milieu réactionnel sous agitation à température ambiante. Après 2 heures, le milieu est versé sur de l'eau (60 ml). Extraire 3 fois avec de l'acétate d'éthyle (30 ml). La phase organique est lavée avec de la saumure, séchée sur sulfate de magnésium, filtrée et concentrée sous pression réduite.

Le brut huileux orangé obtenu est chromatographié sur une colonne de silice en éluant avec un mélange *n-*heptane/acétate d'éthyle 65/35.

Le produit du titre est obtenu sous la forme d'une meringue jaune.

### Stade C : (4-{[({2-Chloro-5-[(2-méthyl-2,3-dihydro-1H-indol-1-yl) carbamoyl]phényl}sulfonyl)amino]méthyl}phényl)acétate de ({[(1Z)-2,2-diéthyl-1-oxidohydrazono]amino}oxy)méthyle

Solubiliser le composé obtenu au Stade B (8,86.10⁻⁴ mol) dans le 1,4-dioxane (5 ml). Placer la solution sous agitation à température ambiante puis ajouter 10 ml d'une solution d'acide chlorhydrique 4 N dans le 1,4-dioxane. Le milieu réactionnel initialement jaunâtre devient de plus en plus foncé jusqu'à être quasiment noir. Après 36 heures d'agitation à température ambiante, le milieu réactionnel est évaporé à sec sous pression réduite. Le résidu noir obtenu est chromatographié sur une colonne de silice en éluant avec un mélange *n-*heptane/acétate d'éthyle 60/40.

Le produit du titre est obtenu sous la forme d'une meringue légèrement jaune.

| *Microanalyse élémentaire :* | *C* | *H* | *N* | *S* |
|---|---|---|---|---|
| *% théorique :* | *54,66* | *5,35* | *12,75* | *4,86* |
| *% expérimental :* | *54, 60* | *5,43* | *12,30* | *4,96* |

### Exemple 13 : 4-{2-[({2-Chloro-5-[(2-méthyl-2,3-dihydro-1H-indol-1-yl)carbamoyl] phényl}sulfonyl)amino]éthyl}benzoate de ({[(1Z)-2,2-diéthyl-1-oxidohydrazono]amino}oxy)méthyle

### Stade A : 4-{2-[(tert-Butoxycarbonyl)({2-chloro-5-[(2-méthyl-2,3-dihydro-1H-indol-1-yl)carbamoyl]phényl}sulfonyl)amino]éthyl}benzoate de méthyle

Solubiliser le composé obtenu au Stade A de l'Exemple 1 (1,04.10⁻² mol) dans l'acétonitrile (20 ml) et placer la solution sous agitation. Ajouter la diisopropyléthylamine (1,24.10⁻² mol) puis après 10 minutes d'agitation, l'intermédiaire 9 (2,20.10⁻² mol) solubilisé dans l'acétonitrile (20 ml). Chauffer le milieu à 60 °C. Après 5 jours, le milieu réactionnel est évaporé à sec sous pression réduite. Le résidu obtenu est chromatographié sur une colonne de silice en éluant avec un mélange *n-*heptane/acétate d'éthyle 75/25.

Le produit du titre est obtenu sous la forme d'une meringue jaune pâle.

### Stade B : Acide 4-{2-[(tert-butoxycarbonyl)({2-chloro-5-[(2-méthyl-2,3-dihydro-1H-indol-1-yl)carbamoyl]phényl}sulfonyl)amino]éthyl} benzoïque

Solubiliser le composé obtenu au Stade A (5,10.10⁻⁴ mol) dans un mélange acétonitrile (18 ml) et eau (3 ml). Ajouter de la lithine (5,10.10⁻³ mol) et chauffer à 55 °C sous agitation. Après 4 heures de chauffage, le milieu réactionnel marron foncé est versé sur de l'eau (100 ml). Extraire 3 fois avec de l'acétate d'éthyle (75 ml). La phase organique est ensuite lavée avec de l'eau et de la saumure, séchée sur sulfate de magnésium, filtrée et évaporée à sec sous pression réduite.

Le produit du titre est obtenu sous la forme d'une meringue marron utilisée sans purification ultérieure.

### Stade C : 4-{2-[(tert-Butoxycarbonyl)({2-chloro-5-[(2-méthyl-2,3-dihydro-1H-indol-1-yl)carbamoyl]phényl}sulfonyl)amino]éthyl}benzoate de ({[(1Z)-2,2-diéthyl-1-oxidohydrazono]amino}oxy)méthyle

Solubiliser le composé obtenu au Stade B (4,0.10⁻⁴ mol) dans le diméthylformamide (3 ml). Placer sous agitation et sous azote et ajouter une solution de *N-*[(*Z*)-(chlorométhoxy)-*NNO*-azoxy]-*N-*éthyléthanamine (5,21.10⁻⁴ mol) dans le diméthylformamide (2 ml). Additionner ensuite en une fois du carbonate de césium (4,20.10⁻⁴ mol). Après 1 heure, le milieu réactionnel est versé sur de l'eau (50 ml). Extraire 3 fois avec de l'acétate d'éthyle (50 ml). La phase organique est lavée avec de l'eau puis de la saumure. Elle est ensuite séchée sur sulfate de magnésium, filtrée puis évaporée à sec sous pression réduite. Le résidu huileux marron obtenu est purifié par chromatographie sur colonne de silice en éluant avec un mélange *n-*heptane/acétate d'éthyle 65/35.

Le produit du titre est obtenu sous la forme d'une meringue marron-orangée.

### Stade D : 4-{2-[({2-Chloro-5-[(2-méthyl-2,3-dihydro-1H-indol-1-yl) carbamoyl]phényl}sulfonyl)amino]éthyl}benzoate de ({[1Z)-2,2-diéthyl-1-oxidohydrazono]amino}oxy)méthyle

Solubiliser le composé obtenu au Stade C (2,17.10⁻⁴ mol) dans le 1,4-dioxane (2 ml). Placer la solution sous agitation à température ambiante puis ajouter 4 ml d'une solution d'acide chlorhydrique 4 N dans le 1,4-dioxane. Le milieu réactionnel initialement jaunâtre devient de plus en plus foncé jusqu'à être quasiment noir. Après 40 heures d'agitation à température ambiante, le milieu réactionnel est évaporé à sec sous pression réduite. Le résidu noir obtenu est chromatographié sur une colonne de silice en éluant avec un mélange *n-*heptane/acétate d'éthyle 55/45. Une deuxième chromatographie en phase inverse sur colonne de Lichroprep RP-18 est nécessaire. Les conditions d'élution sont les suivantes: (éluant A: 1000 ml H₂O/25 ml CH₃CN ; éluant B : 25 ml H₂O/1000 ml CH₃CN) 10 % de B pendant 20 minutes, de 10 à 80 % de B en 70 minutes, de 80 à 100 % de B en 15 minutes.

Le produit du titre est obtenu sous la forme d'une meringue légèrement jaune.

| *Microanalyse élémentaire :* | *C* | *H* | *N* | *S* |
|---|---|---|---|---|
| *% théorique :* | *54, 66* | *5,35* | *12,75* | *4,86* |
| *% expérimental :* | *54,34* | *5,43* | *12,46* | *4,72* |

### Exemple 14 : 4-{2-[({2-Chloro-5-[((2R)-2-méthyl-2,3-dihydro-1H-indol-1-yl) carbamoyl]phényl}sulfonyl)amino]éthyl}benzoate de ({[(1Z)-2,2-diéthyl-1-oxidohydrazono]amino}oxy)méthyle

Le produit du titre est obtenu à partir de l'intermédiaire 8 selon le procédé décrit dans les stades A à D de l'Exemple 13.

### Exemple 15 : 4-{(1-[({2-Chloro-5-[(2-méthyl-2,3-dihydro-1H-indol-1-yl)carbamoyl] phényl}sulfonyl)amino]éthyl}benzoate de ({[(1Z)-2,2-diéthyl-1-oxidohydrazono]amino}oxy)méthyle

### Stade A : 4-{1-[(tert-Butoxycarbonyl)({2-chloro-5-[(2-méthyl-2,3-dihydro-1H-indol-1-yl)carbamoyl]phényl}sulfonyl)amino]éthyl}benzoate de méthyle

Solubiliser le composé obtenu au Stade A de l'Exemple 1 (2,42.10⁻³ mol) dans l'acétonitrile (8 ml) et placer la solution sous agitation. Ajouter la diisopropyléthylamine (2,90.10⁻³ mol) puis après 10 minutes d'agitation, l'intermédiaire 10 (3,38.10⁻³ mol) solubilisé dans l'acétonitrile (2 ml). Chauffer le milieu à 60 °C. Après 3 jours, le milieu réactionnel est évaporé à sec sous pression réduite. Le résidu huileux jaune obtenu est chromatographié sur une colonne de silice en éluant avec un mélange *n-*heptane/acétate d'éthyle 75/25.

Le produit du titre est obtenu sous la forme d'une meringue jaune pâle.

### Stade B : Acide 4-{1-[(tert-butoxycarbonyl)({2-chloro-5-[(2-méthyl-2,3-dihydro-1H-indol-1-yl)carbamoyl]phényl}sulfonyl)amino]éthyl} benzoïque

Solubiliser le composé obtenu au Stade A (1,58.10⁻³ mol) dans un mélange acétonitrile (60 ml) et eau (10 ml). Ajouter de la lithine (1,58.10⁻² mol) et chauffer à 55°C sous agitation. Après 4 heures de chauffage, le milieu réactionnel marron foncé est versé sur de l'eau (200 ml). Extraire 3 fois avec de l'acétate d'éthyle (100 ml). La phase organique est ensuite lavée avec de l'eau et de la saumure, séchée sur sulfate de magnésium, filtrée et évaporée à sec sous pression réduite.

Le produit du titre est obtenu sous la forme d'une meringue marron utilisée sans purification ultérieure.

### Stade C : 4-{1-[(tert-Butoxycarbonyl)({2-chloro-5-[(2-méthyl-2,3-dihydro-1H-indol-1-yl)carbamoyl]phényl}sulfonyl)amino]éthyl}benzoate de ({[(1Z)-2,2-diéthyl-1-oxidohydrazono]amino}oxy)méthyle

Solubiliser le composé obtenu au Stade B (1,07.10⁻³ mol) dans le diméthylformamide (6 ml). Placer sous agitation et sous azote et ajouter une solution de *N-*[(*Z*)-(chlorométhoxy)-*NNO*-azoxy]-*N-*éthyléthanamine (1,40.10⁻³ mol) dans le diméthylformamide (2 ml). Additionner ensuite en une fois du carbonate de césium (1,13.10⁻³ mol). Après 1 heure et 30 minutes, le milieu réactionnel est versé sur de l'eau (50 ml). Extraire 3 fois avec de l'acétate d'éthyle (50 ml). La phase organique est lavée avec de l'eau puis de la saumure. Elle est ensuite séchée sur sulfate de magnésium, filtrée puis évaporée à sec sous pression réduite. Le résidu huileux marron obtenu est purifié par chromatographie sur colonne de silice en éluant avec un mélange *n-*heptane/acétate d'éthyle 65/35.

Le produit du titre est obtenu sous la forme d'une meringue orangée.

### Stade D : 4-{1-[({2-Chloro-5-[(2-méthyl-2,3-dihydro-1H-indol-1-yl) carbamoyl]phényl}sulfonyl)amino]éthyl}benzoate de ({[(1Z)-2,2-diéthyl-1-oxidohydrazono]amino}oxy)méthyle

Solubiliser le composé obtenu au Stade C (7,07.10⁻⁴ mol) dans le 1,4-dioxane (7 ml). Placer la solution sous agitation à température ambiante puis ajouter 15 ml d'une solution d'acide chlorhydrique 4 N dans le 1,4-dioxane. Le milieu réactionnel initialement jaunâtre devient de plus en plus foncé jusqu'à être quasiment noir. Après 2 jours d'agitation à température ambiante, le milieu réactionnel est évaporé à sec sous pression réduite. Le résidu noir obtenu est chromatographié sur une colonne de silice en éluant avec un mélange *n-*heptane/acétate d'éthyle 60/40.

Le produit du titre est obtenu sous la forme d'une meringue jaune pâle.

| *Microanalyse élémentaire :* | *C* | *H* | *N* | *S* |
|---|---|---|---|---|
| *% théorique :* | *54, 66* | *5,35* | *12,75* | *4,86* |
| *% expérimental :* | *54,36* | *5,46* | *12, 28* | *4,71* |

### ETUDE PHARMACOLOGIQUE DES PRODUITS DE L'INVENTION

### EXEMPLE A : Effet diurétique

### In vivo

L'effet diurétique est testé sur des rats Wistar éveillés. Les animaux sont mis à jeun 18 h avant l'expérience et mis en diète hydrique 90 minutes avant l'expérience. Le produit à tester est alors administré oralement par gavage, le rat est placé dans une cage à métabolisme et le volume urinaire est mesuré après 6 heures. Le volume est exprimé par rapport au volume mesuré sur un groupe de rats contrôle.

Résultats : l'augmentation de volume urinaire obtenue avec les produits testés est supérieure ou égale à 20 %.

A titre d'exemple, les composés des Exemples 1 et 10 augmentent, respectivement, l'excrétion urinaire de 108 et de 248 % à la dose orale de 10 mg/kg.

### EXEMPLE B : Activité donneur de NO

### In vitro

Les anneaux d'aorte sans endothélium sont utilisés. Après une première contraction induite par du KCl 60 mM pour caractériser la sensibilité de l'anneau et un lavage, une contraction stable est induite par de la noradrenaline (0,1-0,3 µM) en présence ou non d'ODQ. Une gamme de concentration cumulée est réalisée et l'activité du produit à tester est calculée par une IC₅₀ (dose inhibant de 50 % l'effet maximal).

Résultats : les composés selon l'invention présentent un effet relaxant tout à fait significatif avec des IC₅₀ inférieures à 1 µM.

A titre d'Exemple, les composés des Exemples 1 et 10 présentent, respectivement, une IC₅₀ de 0,12 et 0,04 µM.

### In vivo

L' effet donneur de NO de la molécule est évalué par la baisse de pression provoquée sur des rats SD anesthésiés au pentobarbital. Après stabilisation de la pression artérielle, le produit à tester est administré i. v. en doses croissantes.

Résultats : une diminution d'au moins 50 % de la pression artérielle est observées avec les composés de l'invention pour des doses inférieures ou égales à 0,3 mg/kg.

A titre d'exemple, les composés des Exemples 1 et 10 diminuent, respectivement, la pression artérielle de 56 et 55 % à la dose i.v. de 100 µg/kg.

### EXEMPLE C : Composition pharmaceutique

Formule de préparation pour 1000 comprimés dosés à 100 mg de

| | |
|---|---|
| 3-{[({2-chloro-5-[(2-méthyl-5-nitro-2,3-dihydro-1H-indol-1-yl)carbamoyl]phényl} sulfonyl)amino]méthyl}benzoate de ({[(1*Z*)-2,2-diéthyl-1-oxidohydrazono]amino}oxy) | |
| méthyle (Exemple 1) | 100 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule (I) : 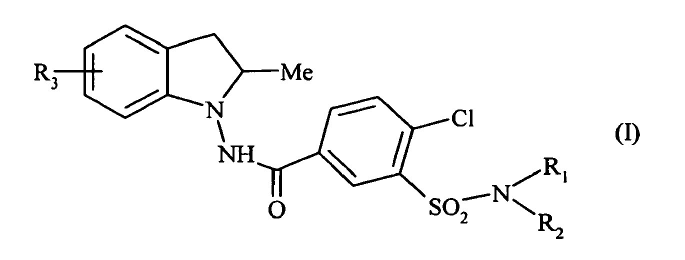 dans laquelle :
R₁ représente un atome d'hydrogène ou un groupement -COOR dans lequel R représente un groupement alkyle (C₁-C₆) linéaire ou ramifié ou arylalkyle (C₁-C₆) linéaire ou ramifié,
R₂ représente un groupement G ou un groupement alkyle (C₁-C₆) linéaire ou ramifié substitué par un groupement G, où G représente un groupement -(CH₂)ₙ-A-(CH₂)ₘ-B-(CR₄R₅)ₚ-(CH₂)ₒ-R₆ dans lequel :
- n vaut 0, 1, 2 ou 3, - m vaut 0, 1, 2 ou 3,
- p vaut 0 ou 1,
- o vaut 0, 1 ou 2,
- R₄ et R₅, identiques ou différents, représentent chacun un atome d'hydrogène ou un groupement alkyle (C₁-C₆) linéaire ou ramifié, étant entendu qu'un des groupements -CH₂- ou -CR₄R₅- de la chaîne G peut également être remplacé, si on le souhaite, par un groupement phénylène, -PhC(O)- ou -PhC(O)O- (où Ph signifie phényle),
- A et B, identiques ou différents, représentent chacun une liaison, un atome d'azote ou un groupement : 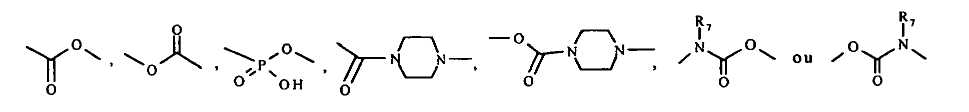 dans lesquels R₇ représente un atome d'hydrogène ou un groupement alkyle (C₁-C₆) linéaire ou ramifié,
- et R₆ représente un groupement 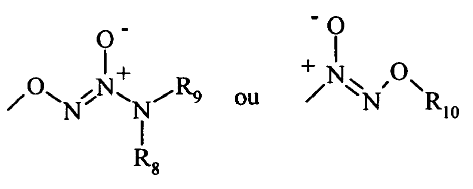 où R₈, R₉ et R₁₀, identiques ou différents, représentent chacun un atome d'hydrogène ou un groupement alkyle (C₁-C₆) linéaire ou ramifié non substitué ou substitué par un groupement amino, ou R₈ et R₉ forment ensemble une chaîne alkylène (C₁-C₆) linéaire ou ramifiée,
R₃ représente un atome d'hydrogène, un groupement alkyle (C₁-C₆) linéaire ou ramifié ou un groupement NO₂,
leurs énantiomères et diastéréoisomères ainsi que leurs sels d'addition à un acide ou à une base pharmaceutiquement acceptable.

2. Composés de formule (I), selon la revendication 1, pour lesquels R₁ représente un atome d'hydrogène, leurs énantiomères et diastéréoisomères ainsi que leurs sels d'addition à un acide ou à une base pharmaceutiquement acceptable.

3. Composés de formule (I), selon la revendication 1, pour lesquels R₃ représente un atome d'hydrogène, leurs énantiomères et diastéréoisomères ainsi que leurs sels d'addition à un acide ou à une base pharmaceutiquement acceptable.

4. Composés de formule (I), selon la revendication 1, pour lesquels R₃ représente un groupement NO₂, leurs énantiomères et diastéréoisomères ainsi que leurs sels d'addition à un acide ou à une base pharmaceutiquement acceptable

5. Composés de formule (I), selon la revendication 1, pour lesquels R₂ représente un groupement : 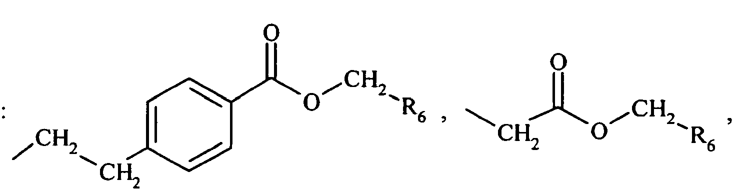 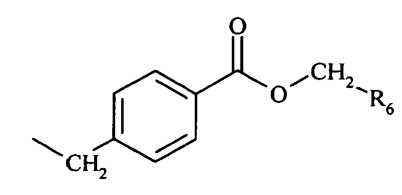 ou —CH₂—R₆ où R₆ est tel que défini dans la
revendication 1, leurs énantiomères et diastéréoisomères ainsi que leurs sels d'addition à un acide ou à une base pharmaceutiquement acceptable.

6. Composés de formule (I), selon la revendication 1, pour lesquels R₆ représente un groupement -O-N=N(O)-NR₈R₉, leurs énantiomères et diastéréoisomères ainsi que leurs sels d'addition à un acide ou à une base pharmaceutiquement acceptable.

7. Composés de formule (I), selon la revendication 1, pour lesquels R₈ et R₉ représentent un groupement alkyle (C₁-C₆) linéaire, leurs énantiomères et diastéréoisomères ainsi que leurs sels d'addition à un acide ou à une base pharmaceutiquement acceptable.

8. Composé de formule (I), selon la revendication 1, qui est le 3-{[({2-chloro-5-[(2-méthyl-5-nitro-2,3-dihydro-1*H-*indol-1-yl)carbamoyl]phényl}sulfonyl)amino]méthyl} benzoate de ({[(1*Z*)-2,2-diéthyl-1-oxidohydrazono]amino}oxy)méthyle, ses énantiomères et diastéréoisomères ainsi que ses sels d'addition à un acide ou à une base pharmaceutiquement acceptable.

9. Composé de formule (I), selon la revendication 1, qui est le 4-{[({2-chloro-5-[(2-méthyl-2,3-dihydro-1*H-*indol-1-yl)carbamoyl]phényl}sulfonyl)amino]méthyl} benzoate de ({[(1*Z*)-2,2-diéthyl-1-oxidohydrazono]amino}oxy)méthyle, ses énantiomères et diastéréoisomères ainsi que ses sels d'addition à un acide ou à une base pharmaceutiquement acceptable.

10. Composé de formule (I), selon la revendication 1, qui est le 4-({[(2-chloro-5-{[(2*R*)-2-méthyl-2,3-dihydro-1*H-*indol-1-yl]carbamoyl}phényl)sulfonyl]amino}méthyl)benzoate de ({[(1*Z*)-2,2-diéthyl-1-oxidohydrazono]amino}oxy)méthyle, ses sels d'addition à un acide ou à une base pharmaceutiquement acceptable.

11. Composé de formule (I), selon la revendication 1, qui est le 4-{2-[({2-chloro-5-[(2-méthyl-2,3-dihydro-1*H-*indol-1-yl)carbamoyl]phényl}sulfonyl)amino]éthyl}benzoate de ({[(1*Z*)-2,2-diéthyl-1-oxidohydrazono]amino}oxy)méthyle, ses énantiomères et diastéréoisomères ainsi que ses sels d'addition à un acide ou à une base pharmaceutiquement acceptable.

12. Composé de formule (I), selon la revendication 1, qui est le 4-{2-[({2-chloro-5-[((2*R*)-2-méthyl-2,3-dihydro-1H-indol-1-yl)carbamoyl]phényl}sulfonyl)amino]éthyl}benzoate de ({[(1*Z*)-2,2-diéthyl-1-oxidohydrazono]amino}oxy)méthyle, ses sels d'addition à un acide ou à une base pharmaceutiquement acceptable.

13. Procédé de préparation des composés de formule (I), selon la revendication 1, **caractérisé en ce que** l'on utilise comme produit de départ le composé de formule (II) : 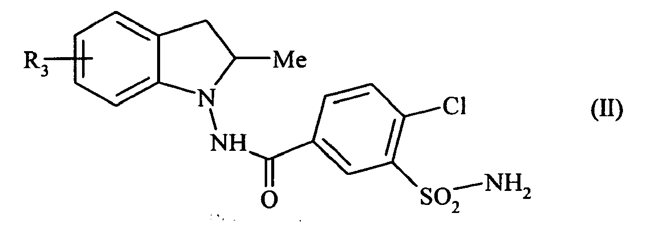 dans laquelle R₃ est tel que défini dans la formule (I),
sur lequel on condense un composé de formule (III) : 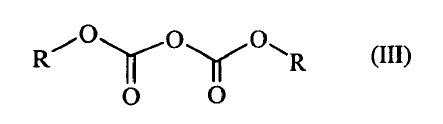 dans laquelle R est tel que défini dans la formule (I),
pour conduire au composé de formule (IV) : 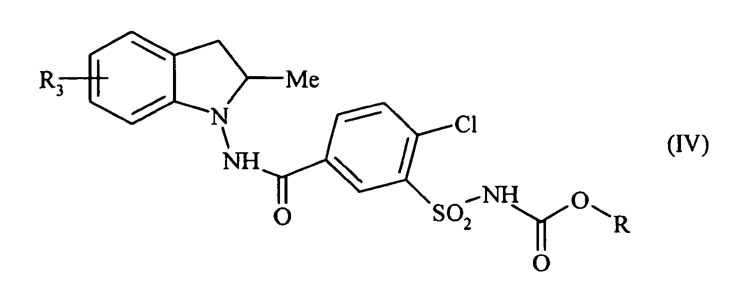 dans laquelle R₃ et R sont tels que définis précédemment,
sur lequel on condense, en présence d'une base, le composé de formule (V) :
X—R₂ (V)
dans laquelle R₂ est tel que défini dans la formule (I), et X représente un atome d'halogène,
pour conduire au composé de formule (I/a), cas particulier des composés de formule (I) : 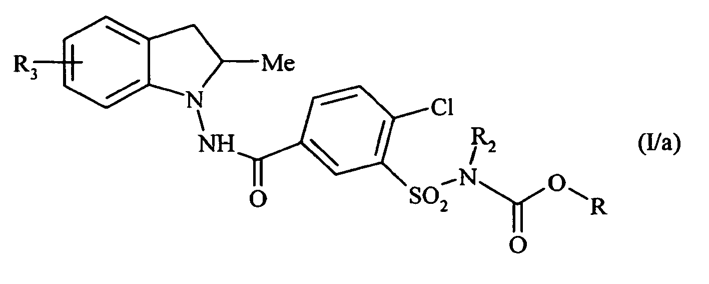 dans laquelle R, R₂ et R₃ sont tels que définis dans la formule (I),
les composés de formule (I/a) pouvant être aussi obtenus par condensation d'un composé de formule Cl-(CH₂)ₒ-R₆, dans laquelle o et R₆ sont tels que définis dans la formule (I), sur une fonction carboxylique ou phosphoramidique présente dans le groupement G,
composés (I/a) qui sont optionnellement chauffés en milieu acide pour conduire au composé de formule (I/b), cas particulier des composés de formule (I) : 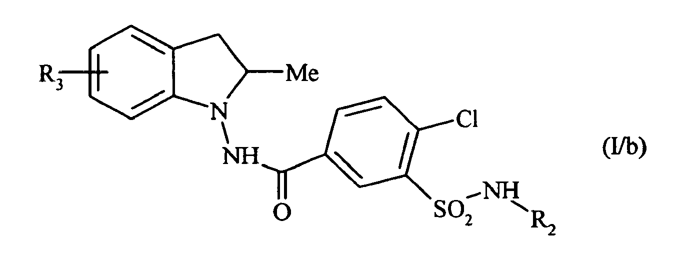 dans laquelle R₂ et R₃ sont tels que définis dans la formule (I),
les composés de formule (I/a) et (I/b) formant l'ensemble des composés de formule (I) que l'on purifie, le cas échéant, selon une technique classique de purification, dont on sépare éventuellement les isomères, selon une technique classique de séparation, et que l'on transforme, si on le souhaite, en ses sels d'addition, à un acide ou à une base pharmaceutiquement acceptable.

14. Compositions pharmaceutiques contenant comme principe actif un composé selon l'une quelconque des revendications 1 à 12 en combinaison avec un ou plusieurs excipients ou véhicules inertes, non toxiques, pharmaceutiquement acceptables.

15. Compositions pharmaceutiques selon la revendication 14 contenant au moins un principe actif selon l'une quelconque des revendications 1 à 12 utiles, en tant que médicament, dans le traitement de l'hypertension et des pathologies cardiovasculaires et de ses complications telles que la rétinopathie, les accidents cérébraux, la démence, l'hypertrophie ventriculaire gauche, l'insuffisance cardiaque, l'angine de poitrine, l'infarctus du myocarde et la néphropathie ; dans le traitement des pathologies cardiovasculaires associées à l'athérothrombose telles que les accidents cérébraux et coronaires, les artérites et les vasculopathies ; dans le traitement des complications vasculaires de nombre de maladies telles que le diabète, l'obésité, le syndrome métabolique, le cancer ou la fibrose du foie ; et dans le traitement des hypertensions d'origine pulmonaire, oculaire ou portale.

## Claims

1. Compounds of formula (I): 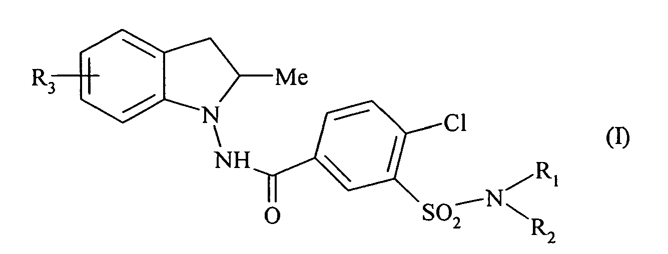 wherein:
R₁ represents a hydrogen atom or a -COOR group wherein R represents a linear or branched (C₁-C₆)alkyl group or an aryl-(C₁-C₆)alkyl group in which the alkyl moiety may be linear or branched,
R₂ represents a group G or a linear or branched (C₁-C₆)alkyl group substituted by a group G, wherein G represents a -(CH₂)ₙ-A-(CH₂)ₘ-B-(CR₄R₅)ₚ-(CH₂)ₒ-R₆ group wherein:
- n is 0, 1, 2 or 3,
- m is 0, 1, 2 or 3,
- p is 0 or 1,
- o is 0, 1 or 2,
- R₄ and R₅, which may be identical or different, each represent a hydrogen atom or a linear or branched (C₁-C₆)alkyl group, wherein one of the groups -CH₂- or -CR₄R₅- of the G chain may equally be replaced, if desired, by a phenylene, -PhC(O)- or -PhC(O)O- group (wherein Ph denotes phenyl),
- A and B, which may be identical or different, each represent a bond, a nitrogen atom or a group: 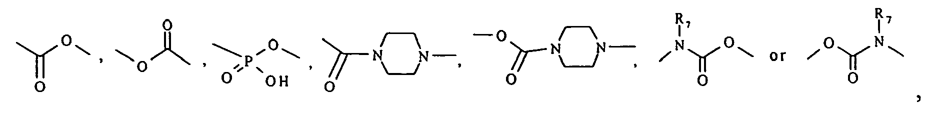 wherein R₇ represents a hydrogen atom or a linear or branched (C₁-C₆)alkyl group,
- and R₆ represents a group 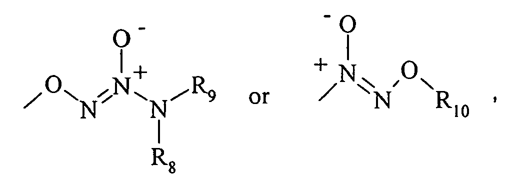 wherein R₈, R₉ and R₁₀, which may be identical or different, each represent a hydrogen atom or a linear or branched (C₁-C₆)alkyl group unsubstituted or substituted by an amino group, or R₈ and R₉ together form a linear or branched (C₁-C₆)alkylene chain,
R₃ represents a hydrogen atom, a linear or branched (C₁-C₆)alkyl group or an NO₂ group,
their enantiomers and diastereoisomers and also addition salts thereof with a pharmaceutically acceptable acid or base.

2. Compounds of formula (I) according to claim 1, wherein R₁ represents a hydrogen atom, their enantiomers and diastereoisomers and also addition salts thereof with a pharmaceutically acceptable acid or base.

3. Compounds of formula (I) according to claim 1, wherein R₃ represents a hydrogen atom, their enantiomers and diastereoisomers and also addition salts thereof with a pharmaceutically acceptable acid or base.

4. Compounds of formula (I) according to claim 1, wherein R₃ represents an NO₂ group, their enantiomers and diastereoisomers and also addition salts thereof with a pharmaceutically acceptable acid or base.

5. Compounds of formula (I) according to claim 1, wherein R₂ represents a group: 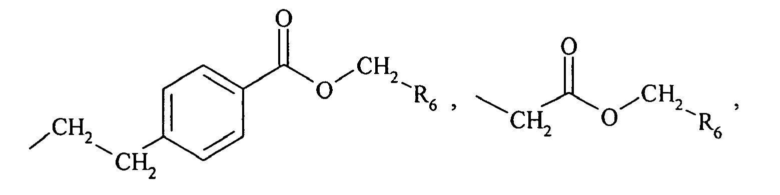 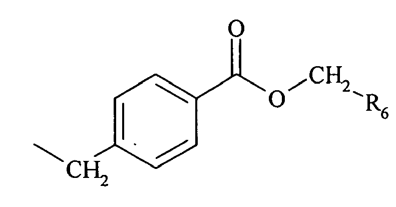 or —CH₂—R₆ wherein R₆ is as defined in
claim 1, their enantiomers and diastereoisomers and also addition salts thereof with a pharmaceutically acceptable acid or base.

6. Compounds of formula (I) according to claim 1, wherein R₆ represents an -O-N=N(O)-NR₈R₉ group, their enantiomers and diastereoisomers and also addition salts thereof with a pharmaceutically acceptable acid or base.

7. Compounds of formula (I) according to claim 1, wherein R₈ and R₉ represent a linear (C₁-C₆)alkyl group, their enantiomers and diastereoisomers and also addition salts thereof with a pharmaceutically acceptable acid or base.

8. Compound of formula (I) according to claim 1, which is ({[(1*Z*)-2,2-diethyl-1-oxidohydrazono]amino}oxy)methyl 3-{[({2-chloro-5-[(2-methyl-5-nitro-2,3-dihydro-1*H-*indol-1-yl)carbamoyl]phenyl}sulphonyl)amino]methyl}benzoate, its enantiomers and diastereoisomers, and also its addition salts with a pharmaceutically acceptable acid or base.

9. Compound of formula (I) according to claim 1, which is ({[(1*Z*)-2,2-diethyl-1-oxido-hydrazono]amino}oxy)methyl 4-{[({2-chloro-5-[(2-methyl-2,3-dihydro-1*H-*indol-1-yl)carbamoyl]phenyl}sulphonyl)amino]methyl}benzoate, its enantiomers and diastereoisomers, and also its addition salts with a pharmaceutically acceptable acid or base.

10. Compound of formula (I) according to claim 1, which is ({[(1Z)-2,2-diethyl-1-oxidohydrazono]amino}oxy)methyl 4-({[(2-chloro-5-{[(2*R*)-2-methyl-2,3-dihydro-1*H-*indol-1-yl]carbamoyl}phenyl)sulphonyl]amino}methyl)benzoate, and its addition salts with a pharmaceutically acceptable acid or base.

11. Compound of formula (I) according to claim 1, which is ({[(1*Z*)-2,2-diethyl-1-oxidohydrazono]amino}oxy)methyl 4-{2-[({2-chloro-5-[(2-methyl-2,3-dihydro-1*H-*indol-1-yl)carbamoyl]phenyl}sulphonyl)amino]ethyl}benzoate, its enantiomers and diastereoisomers, and also its addition salts with a pharmaceutically acceptable acid or base.

12. Compound of formula (I) according to claim 1, which is ({[(1*Z*)-2,2-diethyl-1-oxidohydrazono]amino}oxy)methyl 4-{2-[({2-chloro-5-[((2*R*)-2-methyl-2,3-dihydro-1H-indol-1-yl)carbamoyl]phenyl}sulphonyl)amino]ethyl}benzoate, and its addition salts with a pharmaceutically acceptable acid or base.

13. Process for the preparation of compounds of formula (I) according to claim 1, **characterised in that** there is used as starting material a compound of formula (II): 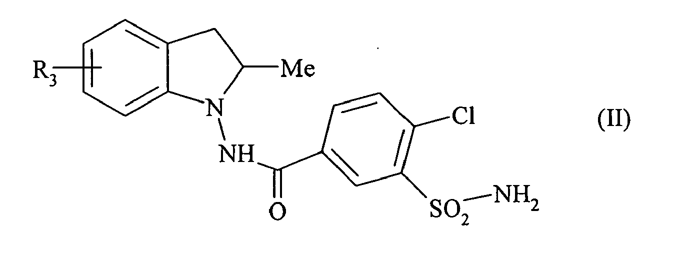 wherein R₃ is as defined for formula (I),
which is condensed with a compound of formula (III): 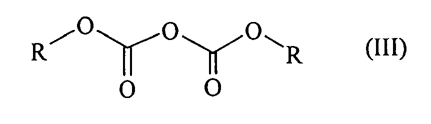 wherein R is as defined for formula (I),
to yield a compound of formula (IV): 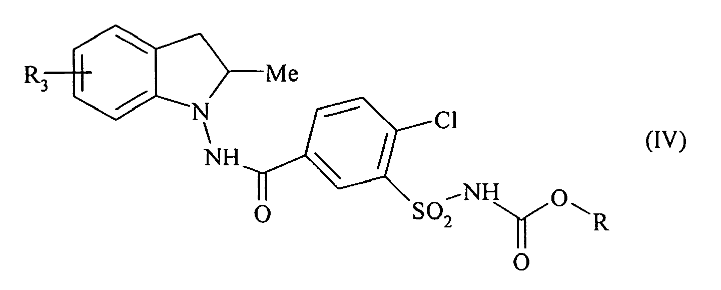 wherein R₃ and R are as defined hereinbefore,
which is condensed, in the presence of a base, with a compound of formula (V):
X-R₂ (V)
wherein R₂ is as defined for formula (I) and X represents a halogen atom,
to yield a compound of formula (I/a), a particular case of the compounds of formula (I): 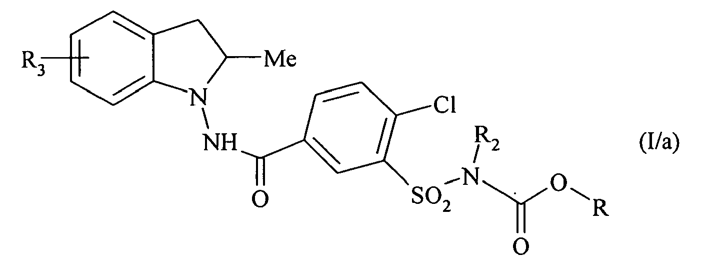 wherein R, R₂ and R₃ are as defined for formula (I),
it being possible for the compounds of formula (I/a) to be obtained also by condensation of a compound of formula Cl-(CH₂)ₒ-R₆, wherein o and R₆ are as defined for formula (I), with a carboxylic or phosphoramidic function present in the group G, which compounds (I/a) are optionally heated in acid medium to yield a compound of formula (I/b), a particular case of the compounds of formula (I): 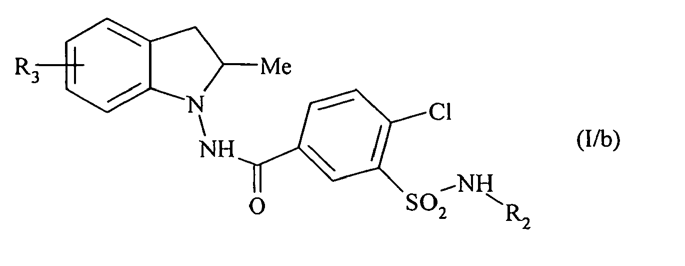 wherein R₂ and R₃ are as defined for formula (I),
which compounds of formulae (I/a) and (I/b), the totality of which constitutes the compounds of formula (I), are purified, where appropriate, according to a conventional purification technique, are optionally separated into isomers according to a conventional separation technique and are converted, if desired, into addition salts with a pharmaceutically acceptable acid or base.

14. Pharmaceutical compositions comprising as active ingredient a compound according to any one of claims 1 to 12 in combination with one or more pharmaceutically acceptable, inert, non-toxic excipients or carriers.

15. Pharmaceutical compositions according to claim 14 comprising at least one active ingredient according to any one of claims 1 to 12 for use, as a medicament, in the treatment of hypertension and cardiovascular pathologies and complications thereof, such as retinopathy, cerebral accidents, dementia, left ventricular hypertrophy, heart failure, angina pectoris, myocardial infarction and nephropathy; in the treatment of cardiovascular pathologies associated with atherothrombosis, such as cerebral and coronary accidents, arteritis and vasculopathies; in the treatment of vascular complications of a number of disorders such as diabetes, obesity, metabolic syndrome, cancer or fibrosis of the liver; and in the treatment of hypertension of pulmonary, ocular or portal origin.

## Patentansprüche

1. Verbindungen der Formel (I): 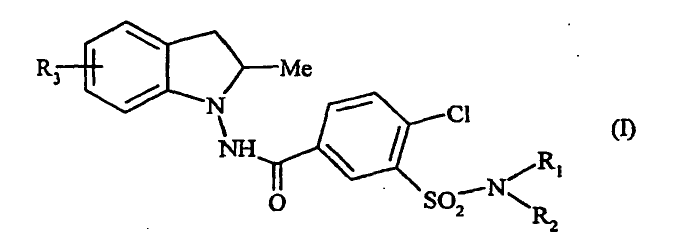 in der:
R₁ ein Wasserstoffatom oder eine -COOR-Gruppe darstellt, in der R eine geradkettige oder verzweigte (C₁-C₆)-Alkylgruppe oder eine geradkettige oder verzweigte Aryl- (C₁-C₆)-alkylgruppe bedeutet,
R₂ eine Gruppe G oder geradkettige oder verzweigte (C₁-C₆)-Alkylgruppe darstellt, die durch eine Gruppe G substituiert ist, oder G eine Gruppe -(CH₂)ₙ-A-(CH₂)ₘ- B-(CR₄R₅)ₚ-(CH₂)ₒ-R₆) darstellt, in der:
- n 0, 1, 2 oder 3 bedeutet, - m 0, 1, 2 oder 3 bedeutet,
- p 0 oder 1 bedeutet,
- o 0, 1 oder 2 bedeutet,
- R₄ und R₅, die identisch oder verschieden sind, jeweils ein Wasserstoffatom oder eine geradkettige oder verzweigte (C₁-C₆)-Alkylgruppe bedeuten, mit der Maßgabe, dass eine der Gruppen -CH₂- oder -CR₄R₅- der Kette G ge- wünschtenfalls ersetzt sein kann durch eine Phenylengruppe oder eine Gruppe - PhC(O)- oder eine Gruppe -PhC(O)O- (worin Ph für Phenyl steht),
- A und B identisch oder verschieden sind und jeweils eine Bindung, ein Stick- stoffatom oder eine Gruppe darstellen: 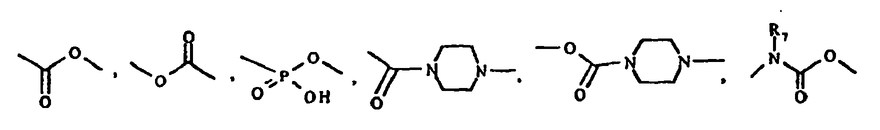 oder 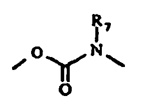 in denen R₇ ein Wasserstoffatom oder eine geradkettige oder verzweigte (C₁- C₆)-Alkylgruppe bedeutet,
- und R₆ eine Gruppe 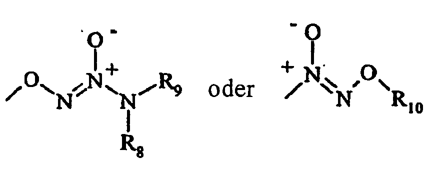 darstellt, worin R₈, R₉ und R₁₀, die identisch oder verschieden sind, jeweils ein Wasserstoffatom oder eine geradkettige oder verzweigte (C₁-C₆)-Alkylgruppe, die nichtsubstituiert oder durch eineAminogruppe substituiert ist, bedeuten oder R₈ und R₉ gemeinsam eine geradkettige oder verzweigte (C₁-C₆)-Alkylenkette bilden, und
R₃ ein Wasserstoffatom, eine geradkettige oder verzweigte (C₁-C₆)-Alkylgruppe oder eine NO₂-Gruppe darstellt,
deren Enantiomeren und Diastereoisomeren sowie deren Additionssalze mit einer pharmazeutisch annehmbaren Säure oder Base.

2. Verbindungen der Formel (I) nach Anspruch 1, worin R₁ ein Wasserstoffatom bedeutet, deren Enantiomeren und Diastereoisomeren sowie deren Additionssalze mit einer pharmazeutisch annehmbaren Säure oder Base.

3. Verbindungen der Formel (I) nach Anspruch 1, worin R₃ ein Wasserstoffatom bedeutet, deren Enantiomeren und Diastereoisomeren sowie deren Additionssalze mit einer pharmazeutisch annehmbaren Säure oder Base.

4. Verbindungen der Formel (I) nach Anspruch 1, worin R₃ eine NO₂-Gruppe bedeutet, deren Enantiomeren und Diastereoisomeren sowie deren Additionssalze mit einer pharmazeutisch annehmbaren Säure oder Base.

5. Verbindungen der Formel (I) nach Anspruch 1, worin R₂ eine Gruppe der Formeln: 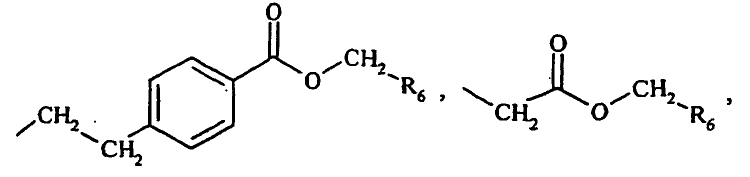 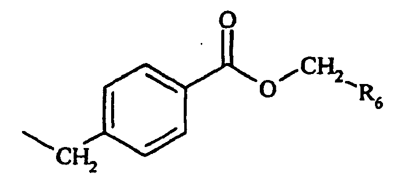 oder -CH₂-R₆ bedeutet, worin R₆ die in Anspruch 1 angegebenen Bedeutungen besitzt, deren Enantiomeren und Diastereoisomeren sowie deren Additionssalze mit einer pharmazeutisch annehmbaren Säure oder Base.

6. Verbindungen der Formel (I) nach Anspruch 1, worin R₆ eine Gruppe der Formel -O-N=N(O)-NR₈R₉ bedeutet, deren Enantiomeren und Diastereoisomeren sowie deren Additionssalze mit einer pharmazeutisch annehmbaren Säure oder Base.

7. Verbindungen der Formel (I) nach Anspruch 1, worin R₈ und R₉ eine geradkettige (C₁-C₆)-Alkylgruppe bedeutet, deren Enantiomeren und Diastereoisomeren sowie deren Additionssalze mit einer pharmazeutisch annehmbaren Säure oder Base.

8. Verbindung der Formel (I) nach Anspruch 1, nämlich 3-{[({2-Chlor-5-[(2-methyl-5-nitro-2,3-dihydro-1*H-*indol-1-yl)-carbamoyl]-phenyl}-sulfonyl)-amino]-methyl}-benzoesäure-({[(1*Z*)-2,2-diethyl-1-oxidohydrazono]-amino}-oxy)-methylester, deren Enantiomeren und Diastereoisomeren sowie deren Additionssalze mit einer pharmazeutisch annehmbaren Säure oder Base.

9. Verbindung der Formel (I) nach Anspruch 1, nämlich 4-{[({2-Chlor-5-[(2-methyl-2,3-dihydro-1*H-*indol-1-yl)-carbamoyl]-phenyl}-sulfonyl)-amino]-methyl}-benzoesäure-({[(1*Z*)-2,2-diethyl-1-oxidohydrazono]-amino}-oxy)-methylester, deren Enantiomeren und Diastereoisomeren sowie deren Additionssalze mit einer pharmazeutisch annehmbaren Säure oder Base.

10. Verbindung der Formel (I) nach Anspruch 1, nämlich 4-({[(2-Chlor-5-{[(2*R*)-2-methyl-2,3-dihydro-1*H*-indol-1-yl]-carbamoyl}-phenyl)-sulfonyl]-amino}-methyl)-benzoesäure-({[(1*Z*)-2,2-diethyl-1-oxidohydrazono]-amino}-oxy)-methylester sowie deren Additionssalze mit einer pharmazeutisch annehmbaren Säure oder Base.

11. Verbindung der Formel (I) nach Anspruch 1, nämlich 4-{2-[({2-Chlor-5-[(2-methyl-2,3-dihydro-1*H-*indol-1-yl)-carbamoyl]-phenyl}-sulfonyl)-amino]-ethyl}-benzoesäure-({[(1*Z*)-2,2-diethyl-1-oxidohydrazono]-amino}-oxy)-methylester, deren Enantiomeren und Diastereoisomeren sowie deren Additionssalze mit einer pharmazeutisch annehmbaren Säure oder Base.

12. Verbindung der Formel (I) nach Anspruch 1, nämlich 4-{2-[({2-Chlor-5-[((2*R*)-2-methyl-2,3-dihydro-1*H*-indol-1-yl)-carbamoyl]-phenyl}-sulfonyl)-amino]-ethyl}-benzoesäure-({[(1*Z*)-2,2-diethyl-1-oxidohydrazono]-amino}-oxy)-methylester sowie deren Additionssalze mit einer pharmazeutisch annehmbaren Säure oder Base.

13. Verfahren zur Herstellung der Verbindungen der Formel (I) nach Anspruch 1, **dadurch gekennzeichnet, dass** man als Ausgangsprodukt die Verbindung der Formel (II): 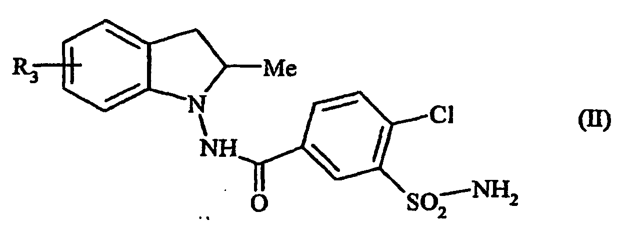 in der R₃ die bezüglich der Formel (I) angegebenen Bedeutungen besitzt, verwendet, welche man mit einer Verbindung der Formel (III): 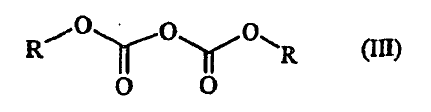 in der R die bezüglich der Formel (I) angegebenen Bedeutungen besitzt, kondensiert, zur Bildung der Verbindung der Formel (IV): 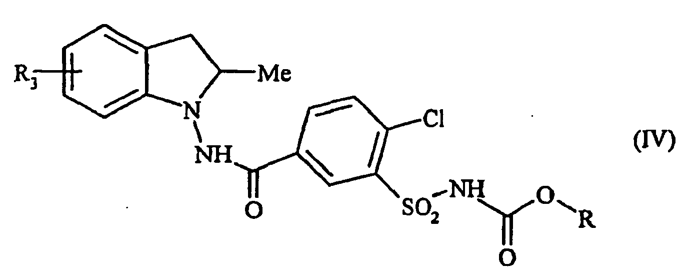 in der R₃ und R die oben angegebenen Bedeutungen besitzen,
welche man in Gegenwart einer Base mit der Verbindung der Formel (V):
X - R₂ (V)
in der R₂ die bezüglich der Formel (I) angegebenen Bedeutungen besitzt und X ein Halogenatom darstellt, kondensiert,
zur Bildung der Verbindung der Formel (I/a), einem Sonderfall der Verbindungen der Formel (I): 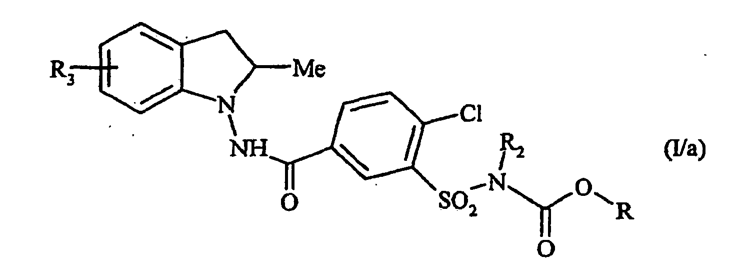 in der R, R₂ und R₃ die bezüglich der Formel (I) angegebenen Bedeutungen besitzen, welche Verbindungen der Formel (I/a) auch erhalten werden können durch Kondensation einer Verbindung der Formel Cl-(CH₂)ₒ-R₆, worin o und R₆ die bezüglich der Formel (I) angegebenen Bedeutungen besitzen, auf die Carboxylfunktion oder Phosphoramidfunktion, die in der Gruppe G vorhanden ist,
welche Verbindungen der Formel (I/a) gegebenenfalls in einem sauren Medium erhitzt werden zur Bildung der Verbindung der Formel (I/b), einem Sonderfall der Verbindungen der Formel (I): 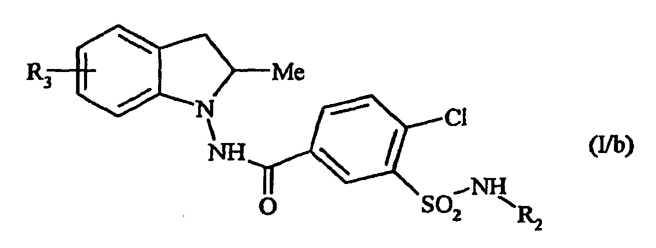 in der R₂ und R₃ die bezüglich der Formel (I) angegebenen Bedeutungen besitzen, welche Verbindungen der Formeln (I/a) und (I/b), welche die Gesamtheit der Verbindungen der Formel (I) bilden, gegebenenfalls mit Hilfe einer klassischen Reinigungsmethode gereinigt werden, welche man gegebenenfalls mit Hilfe einer klassischen Trennungsmethode in ihre Isomeren auftrennt und welche man gewünschtenfalls mit einer pharmazeutisch annehmbaren Säure oder Base in ihre Additionssalze überführt.

14. Pharmazeutische Zubereitungen enthaltend als Wirkstoff eine Verbindung nach einem der Ansprüche 1 bis 12 in Kombination mit einem oder mehreren inerten, nichttoxischen, pharmazeutisch annehmbaren Hilfsstoffen oder Trägermaterialien.

15. Pharmazeutische Zubereitungen nach Anspruch 14, enthaltend mindestens einen Wirkstoff nach einem der Ansprüche 1 bis 12, nützlich als Arzneimittel zur Behandlung der Hypertension und von kardiovaskulären Erkrankungen und ihren Komplikationen, wie Retinopathie, Schlaganfälle, Demenz, Hypertrophie des linken Ventrikels, Herzinsuffizienz, Angina pectoris, Myokardinfarkt und Nephropathie; für die Behandlung von kardiovaskulären Erkrankungen, die mit der Atherothrombose verknüpft sind, wie Schlaganfälle und Infarkte, Arteriitis und Gefäßerkrankungen; für die Behandlung von Gefäßkomplikationen einer Vielzahl von Erkrankungen, wie Diabetes, Fettsucht, Stoffwechselsyndrome, Krebs oder Leberfibrose; und für die Behandlung von Hypertensionen pulmonalen, okularen oder portalen Ursprungs.
